(19) **Europäisches Patentamt**
**European Patent Office**
**Office européen des brevets**

(11) **EP 2 062 047 B1**

(12) **FASCICULE DE BREVET EUROPEEN**

(45) Date de publication et mention
de la délivrance du brevet:
**09.03.2011 Bulletin 2011/10**

(51) Int Cl.:
*G01N 33/50* *(2006.01)*     *G01N 33/577* *(2006.01)*

(21) Numéro de dépôt: **07825673.2**

(86) Numéro de dépôt international:
**PCT/IB2007/003498**

(22) Date de dépôt: **12.09.2007**

(87) Numéro de publication internationale:
**WO 2008/032217 (20.03.2008 Gazette 2008/12)**

(54) **METHODE D'INVESTIGATION DE LA REPONSE A UN TRAITEMENT PAR UN ANTICORPS MONOCLONAL**

VERFAHREN ZUR UNTERSUCHUNG DER REAKTION AUF EINE BEHANDLUNG MIT EINEM MONOKLONALEN ANTIKÖRPER

METHOD FOR INVESTIGATING THE RESPONSE TO A TREATMENT WITH A MONOCLONAL ANTIBODY

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **13.09.2006  FR 0607990**
**15.12.2006  EP 06291942**
**23.01.2007  FR 0700456**

(43) Date de publication de la demande:
**27.05.2009  Bulletin 2009/22**

(73) Titulaires:
- **Glycode**
  **19140 Uzerche (FR)**
- **Universite Francois Rabelais de Tours**
  **37041 Tours Cedex 1 (FR)**

(72) Inventeurs:
- **THIBAULT, Gilles**
  **37510 Ballan-Miré (FR)**
- **WATIER, Hervé**
  **37510 Ballan-Miré (FR)**

- **DALL'OZZO, Sébastien**
  **Addlestone Surrey KT15 1LF (GB)**

(74) Mandataire: **Marcadé, Véronique et al**
**Cabinet Ores**
**36, rue de St Pétersbourg**
**75008 Paris (FR)**

(56) Documents cités:
**EP-A1- 1 298 219     WO-A-01/77181**
**FR-A- 2 844 513     FR-A1- 2 844 521**

- **SHIELDS R L ET AL: "High resolution mapping of the binding site on human IgG1 for FcgammaRI, FcgammaRII, FcgammaRIII, and FcRn and design of IgG1 variants with improved binding to the FcgammaR" JOURNAL OF BIOLOGICAL CHEMISTRY, AMERICAN SOCIETY OF BIOLOCHEMICAL BIOLOGISTS, BIRMINGHAM,, US, vol. 276, no. 9, 2 mars 2001 (2001-03-02), pages 6591-6604, XP002271092 ISSN: 0021-9258**

**Description**

La présente invention concerne le domaine des anticorps monoclonaux (AcMo), en particulier celui des anticorps monoclonaux recombinants (AcMor) utilisés comme médicaments. Plus spécifiquement, l'invention se rapporte aux étapes de mise au point ou de contrôle qualité de ces anticorps, ainsi qu'à la sélection des patients susceptibles de répondre efficacement à un traitement par un anticorps monoclonal donné.

La bio-ingénierie des AcMor a permis le développement de médicaments tels que le rituximab, l'infliximab ou le trastuzumab, ciblant respectivement le CD20, le TNFα et l'antigène ErbB2, et qui constituent des progrès thérapeutiques majeurs notamment en cancérologie et dans les maladies inflammatoires systémiques. Cependant, bien qu'ils soient conçus sur le modèle des anticorps naturellement produits par l'organisme, il existe une importante variabilité de la réponse thérapeutique et des effets indésirables entre les patients. Une partie de la variabilité de réponse n'est pas liée à la maladie elle-même (cancer, polyarthrite rhumatoïde, maladie de Crohn,...), mais à des facteurs individuels, tels que la constitution génétique du patient. Il est donc important d'analyser les mécanismes impliqués dans la réponse à un AcMo, afin d'optimiser ces traitements.

Un anticorps (Ac) est une molécule bi-fonctionnelle constituée d'une portion Fab permettant la liaison à l'antigène (Ag) dont il est spécifique, et d'une portion Fc capable de recruter après liaison à l'Ag des molécules actives (système du complément) ou des cellules exprimant des récepteurs (FcRs) pour cette portion. Ces molécules et cellules exercent alors des fonctions effectrices comme la cytotoxicité dépendante du complément (CDC) ou la cytotoxicité dépendante des Ac *(antibody dependent cellular cytotoxicity :* ADCC), qui permettent l'élimination de l'Ag ou de la cellule exprimant l'Ag (effet recherché dans les traitements anti-cancéreux par exemple). La liaison de l'Ac sur l'Ag, qui est indépendante de sa portion Fc, peut suffire pour neutraliser certains d'entre eux voire pour déclencher l'apoptose de la cellule qui l'exprime.

Les AcMor thérapeutiques peuvent agir par le biais de ces différents mécanismes, mais la part relative de chacun d'eux est le plus souvent méconnue. Des travaux menés chez la souris ont permis de montrer que les mécanismes dépendant de l'interaction de la portion Fc de. l'AcMor avec les FcRs étaient impliqués dans l'activité d'AcMor cytolytiques (détruisant des cellules) *in vivo.* Il a également été montré que la réponse clinique de patients atteints de lymphomes malins non Hodgkiniens au rituximab dépendait du polymorphisme du gène *FCGR3A* (Cartron et al., 2002). Ce gène code le FcγRIIIa (aussi désigné CD16a), un FcR de faible affinité pour les immunoglobulines (Ig) G, exprimé principalement sur les cellules tueuses naturelles *(natural killer :* NK) et les macrophages, responsables de l'ADCC. Le polymorphisme se traduit par la présence d'une valine (V) ou d'une phénylalanine (F) en position 158 du FcγRIIIa. L'influence du polymorphisme 158-V/F a été confirmée par d'autres équipes pour ce même AcMor y compris dans d'autres hémopathies malignes, mais aussi pour d'autres AcMor. Dall'Ozzo *et al.* ont également démontré que la faculté des cellules NK à exercer l'ADCC dépendait du polymorphisme de *FCGR3A :* elle est plus importante quand les cellules expriment uniquement l'allotype 158-V qui possède une meilleure affinité pour le Fc des AcMor (Dall'Ozzo et al., 2004). Ces résultats démontrent que des mécanismes d'action dépendant du FcγRIIIa et des cellules exprimant ce récepteur, en particulier les cellules NK, sont fortement impliqués dans l'activité thérapeutique de ces AcMor chez l'Homme.

Les cellules NK font partie de la classe des grands lymphocytes granuleux *(LGL : Large granular lymphocyte).* Elles participent au système de défense immunologique non spécifique, vis-à-vis des cellules tumorales, des virus, des bactéries à développement intracellulaire, et des cellules allogéniques (Trinchieri, 1989). Ces cellules, généralement identifiées par l'expression membranaire du CD56 et l'absence d'expression de la molécule CD3, représentent 5 à 20% des lymphocytes. Comme leur nom l'indique, elles exercent une activité cytotoxique sans immunisation préalable. Cette cytotoxicité, dite naturelle, est déclenchée par la stimulation de récepteurs activateurs dont les ligands sont exprimés sur les cellules cibles ayant subi un stress, comme une transformation tumorale ou l'infection par un virus. Comme mentionné ci-dessus, les cellules NK exercent également l'ADCC, qui est déclenchée par l'engagement du FcγRIIIa/CD16a (exprimé par environ 95% d'entre elles) par la portion Fc des anticorps spécifiques de la cellule cible et fixés sur cette dernière. L'implication de l'ADCC dans les mécanismes d'action des AcMor thérapeutiques cytolytiques est maintenant bien établie (Dall'Ozzo et al., 2004; Greenwood et al., 1993; Reff et al., 1994). Ces deux mécanismes de déclenchement conduisent à l'activation de la cellule NK, puis à sa dégranulation, et ainsi au relargage de son contenu cytolytique : principalement composé de granzymes et de perforine, qui ont une activité protéasique et pro-apoptotique sur les cellules cibles (Smyth et al., 2005). Ces granules contiennent également au niveau de leur membrane des glycoprotéines fortement N-glycosylées, faisant parties de la famille des LAMP *(Lysosome Associated Membrane Protein),* qui comprennent LAMP-1 (CD107a), LAMP-2 (CD107b) et LAMP-3 (CD63) (Fukuda, 1991), qui ne sont pas exprimés sur la membrane plasmique. Durant le phénomène de dégranulation, les cellules effectrices libèrent leur perforine et les molécules LAMP sont redistribuées de manière concomitante sur la membrane plasmique de la cellule cytotoxique (Betts et al., 2003). Elles deviennent alors accessibles au marquage par des AcMo fluorescents et détectables par cytométrie en flux. L'expression membranaire de CD107 permet ainsi d'identifier les cellules ayant dégranulé (Betts *et al.,* 2003) et exercé la cytotoxicité (Bryceson *et al.,* 2005). La détection du CD107 peut donc représenter une alternative aux tests de cytotoxité classiques basés sur la libération par les cellules cibles lysées de marqueurs, comme le $^{51}$Cr,

dont elles ont été préalablement chargées (Brunner et al., 1968).

Les cellules NK produisent également des cytokines avec les deux mécanismes de déclenchement décrits ci-dessus : naturel ou dépendant des Ac (*antibody dependent cytokine production :* ADCP). Cette sécrétion se compose principalement d'interféron gamma (IFNγ), de facteur de nécrose des tumeurs (*tumor necrosis factor* alpha : TNFα) et de GM-CSF (Trinchieri, 1989). Cette production de cytokines participe au recrutement des autres médiateurs de l'inflammation sur les sites d'infection ou de croissance tumorale. L'IFNγ permet le recrutement des macrophages, l'augmentation de l'expression des molécules de CMH de classe I et II, le développement de la réponse immunitaire spécifique, et la synthèse d'autres cytokines influençant l'orientation des lymphocytes T vers une réponse de type Th1 (Boehm et al., 1997; Gajewski et al., 1988; Nathan et al., 1984). Différentes hypothèses ont été proposées pour expliquer les mécanismes qui orientent les cellules NK vers la cytotoxicité ou la sécrétion de cytokines déclenchée selon le mécanisme naturel : l'existence de deux sous populations NK distinctes (Cooper et al., 2001), la nature de la cellule cible (Kurago et al., 1998), la stimulation de co-récepteurs cellulaires (Colucci et al., 2001; Rajagopalan et al., 2001). Ces résultats sont à rapprocher de ceux rapportés par Faroudi *et al.* montrant que la réponse cytokinique des lymphocytes T CD8+ nécessite un seuil d'activation supérieur à celui de la cytotoxicité (Faroudi et al., 2003).

L'ADCP a été relativement peu étudiée. Anegon *et al.* ont montré que la stimulation des cellules NK par un anti-CD16 entraînait l'accumulation d'ARNm de l'IFNγ et du TNFα (Anegon et al., 1988). Cependant, la sécrétion des cytokines correspondantes nécessite habituellement la co-stimulation des cellules NK par des cytokines comme l'IL-2, l'IL-12 et l'IL-15 (Colucci et al., 2001; Huntington et al., 2005; Parihar et al., 2002). De même, des cellules NK mises en présence de cellules cibles SKBR-3 sensibilisées par le trastuzumab, un AcMo thérapeutique dirigé contre l'antigène ErbB2 exprimé par ces cellules, n'exercent l'ADCP qu'en présence d'IL-2 ou d'IL12 (Parihar et al., 2002), alors qu'elles exercent l'ADCC en absence de ces cytokines (Cooley et al., 1999). Les deux fonctions ne sont donc pas toujours régulées *in vitro* de manière coordonnée. Il a été proposé, qu'*in vivo*, l'engagement du FcγRIIIa par des AcMo thérapeutiques pourrait déclencher une réponse cytokinique (par ADCP) participant à leurs réponses pharmacologiques (Parihar et al., 2002; Wing et al., 1996) ou à des effets indésirables comme le syndrome de libération de cytokines observé au cours de la première perfusion (Wing et al., 1996).

A l'heure actuelle, il apparaît nécessaire d'améliorer l'efficacité des traitements par AcMor. Plusieurs voies sont envisageables pour cela.

La première voie consiste à tenter d'optimiser ou de modifier le médicament lui-même. Il est ainsi possible de générer des AcMor ayant une meilleure affinité pour l'Ag cible, de générer des AcMor dirigés contre un épitope plus pertinent ou de modifier la portion Fc des AcMor de façon à augmenter l'efficacité des fonctions dépendantes de celle-ci. Des variations de la glycosylation de la portion Fc (en faisant produire ces AcMor par des cellules particulières), ou des modifications de la séquence protéique ont ainsi permis d'augmenter l'affinité des AcMor pour des formes solubles de FcγR et au plan fonctionnel d'augmenter l'ADCC exercée *in vitro* en présence de ces AcMor modifiés sur des cellules cibles données exprimant l'Ag (Shields et al., 2001; Shinkawa et al., 2003; Umana et al., 1999). D'autres modifications peuvent aussi conduire à améliorer l'activation du complément. Ces AcMor à région Fc modifiée (AcMor de seconde génération) sont actuellement en phase de développement. Une autre façon d'améliorer les traitements par anticorps serait d'administrer des thérapeutiques adjuvantes permettant d'augmenter les réponses dépendantes du FcγRIIIa, comme cela a déjà été réalisé avec des cytokines comme l'IL-2 ou l'IL-12. Des outils permettant de tester facilement l'effet des modifications apportées à la région Fc et/ou de l'apport d'un adjuvant sur l'activation des cellules effectrices seraient très utiles dans ce contexte. De même, de tels outils permettraient de tester la variabilité (inter-lots) et/ou la stabilité (intra-lot) des propriétés fonctionnelles de la portion Fc dans la phase de production des AcMor.

Le Brevet EP1298219 décrit une méthode pour mesurer l'activité de fragments Fc d'anticorps monoclonaux, en mettant en contact les fragments Fc immobilisés sur une plaque avec des cellules THP-1 exprimant le FCγRIIIa activées par un traitement à l'IFNγ ; après incubation, la réponse cellulaire est observée par mesure de la quantité d'IL-8 produite.

Une autre voie d'amélioration de l'efficacité des traitements par les AcMor consiste à optimiser la sélection des patients susceptibles de recevoir les traitements. L'identification des facteurs génétiques de réponse thérapeutique tels que le polymorphisme de *FCGR3A,* domaine de la pharmacogénétique, peut permettre de mieux sélectionner les patients répondeurs, mais peut aussi permettre d'optimiser le traitement chez les "non-répondeurs", notamment par modification des schémas d'administration, adjonction de traitements synergiques... Cependant, aujourd'hui, la génétique ne permet de classer les patients que dans des groupes statistiquement bons ou mauvais répondeurs. Ainsi, parmi les patients appartenant au groupe génétique mauvais répondeur certains présentent une réponse thérapeutique favorable. Dès lors l'utilisation de tests permettant d'évaluer la qualité de réponse individuelle des cellules effectrices FcγRIIIa+ des patients pourrait être plus prédictive de la réponse thérapeutique

La présente invention propose des outils utilisables dans les trois applications mentionnées ci-dessus (amélioration et contrôle de qualité des AcMor thérapeutiques et sélection des patients), car elle concerne une technologie simple à mettre en oeuvre, reproductible et fiable, permettant de quantifier les fonctions effectrices des cellules exprimant le FcγRIIIa (balance ADCC/ADCP), en réponse aux AcMor. Cette technologie est fondée sur la combinaison de plusieurs moyens, et sur la démonstration qu'il est possible d'analyser de façon simultanée les réponses ADCC et ADCP de cellules effectrices exprimant le FcγRIIIa (en particulier des cellules NK), en incubant ces cellules en présence d'AcMor immobilisés sur un support plastique qui engagent donc le FcγRIIIa en s'affranchissant de toute cible cellulaire. Il devient ainsi possible de comparer la capacité d'AcMor différents (reconnaissant différents Ag sur différentes cibles) à induire l'ADCC et l'ADCP par les cellules NK ou d'autres effecteurs cellulaires.

L'invention porte donc en premier lieu sur une méthode pour évaluer *in vitro* les fonctions effectrices de cellules exprimant le FcγRIIIa, en particulier de cellules NK, en réponse à un anticorps monoclonal à tester, comportant au moins les étapes suivantes :

(i) les cellules exprimant le FcγRIIIa sont mises en contact avec ledit anticorps monoclonal, immobilisé sur un support, en présence d'un agent inhibant la sécrétion des cytokines par lesdites cellules ;
(ii) à titre de contrôle positif de l'activation des cellules exprimant le FcγRIIIa, la même expérience est réalisée en utilisant, à la place de l'anticorps monoclonal à tester, un anticorps monoclonal dirigé contre le récepteur FcγRIIIa ;
(iii) après un temps d'incubation d'au moins 1 heure, la réponse des cellules exprimant le FcγRIIIa est observée en mesurant la présence du marqueur CD107 à la surface cellulaire, ainsi que la présence d'IFNγ intracellulaire et/ou de TNFα intracellulaire.

Dans la méthode ci-dessus, la présence du marqueur CD107 à la surface cellulaire, et l'IFNγ et/ou le TNFα intracellulaires sont de préférence mesurés par cytométrie en flux.

Selon une mise en oeuvre préférée de la méthode de l'invention, l'incubation est effectuée en présence d'un anticorps monoclonal anti-CD107 couplé à un marqueur. Ceci permettra de mesurer, à la fin de l'incubation, la présence du marqueur CD 107 à la surface cellulaire, qui révèle les cellules ayant dégranulé.

Dans une méthode selon l'invention, la mesure de l'IFNγ et/ou du TNFα est de préférence effectuée en perméabilisant les cellules à la fin de l'incubation, puis en utilisant un anticorps monoclonal dirigé contre la cytokine à mesurer, couplé à un marqueur.

Les inventeurs ont par ailleurs confirmé que l'engagement du FcγRIIIa (CD 16) par un anticorps se traduit par une perte d'expression membranaire de cette molécule sur les cellules. La détection du CD 16 à la surface des cellules apporte ainsi une information supplémentaire sur la réponse des cellules à un AcMo donné. Selon une autre mise en oeuvre préférée de la présente invention, la méthode ci-dessus comporte donc également une étape de mesure de l'expression du CD16 à la surface des cellules exprimant le FcγRIIIa, par cytométrie en flux après marquage desdites cellules à la fin de l'incubation, avec un anticorps monoclonal anti-CD16 couplé à un marqueur.

La technique de cytométrie en flux est bien connue de l'homme du métier, qui saura choisir des marqueurs appropriés pour distinguer les différentes molécules à mesurer. En particulier, il est avantageux de choisir, comme marqueurs couplés aux anticorps monoclonaux anti-CD107, anti-IFNγ, anti-TNFα, et anti-CD16, des fluorochromes émettant à des longueurs d'ondes distinctes. A titre d'exemples non limitatifs, on peut citer l'isothiocyanate de fluorescéine (FITC), l'Alexa Fluor 488, l'Alexa Fluor 405, la phycoérythrine (PE), la *peridinin chlorophyll protein (*PerCP), la phycoerythrine cyanine 5 (PC5), la phycoerythrine cyanine 5.5 (PC5.5), la phycoerythrine Texas red® (ECD), la phycoerythrine cyanine 7 (PC7), l'allophycocyanine (APC), l'Alexa Fluor 647, Alexa fluor 700, la phycoerythrine Alexa Fluor 700 (PAF7), l'Alexa fluor 700 et l'APC cyanine 7 (APC7).

De même, l'homme du métier peut choisir n'importe quel agent approprié pour inhiber la sécrétion des cytokines par les cellules exprimant le FcγRIIIa, parmi les composés connus pour inhiber l'exocytose, tels que, par exemple, la Brefeldine A.

L'anticorps monoclonal à tester et l'anticorps monoclonal dirigé contre le récepteur FcγRIIIa utilisé comme contrôle sont de préférence fixés à une concentration permettant de saturer le support sur lequel ils sont adsorbés. Des plaques utilisables pour mettre en oeuvre la méthode de l'invention peuvent notamment être obtenues en les sensibilisant avec une concentration d'environ 1 à 3 μg/ml d'anticorps, pendant au moins 6h, de préférence 8 à 12h ou davantage. Toutefois, dans certaines situations, il peut être préférable d'utiliser des concentrations subsaturantes d'anticorps, par exemple pour déterminer la concentration sensibilisante d'un AcMor donné qui permet d'obtenir une réponse effectrice mesurable des cellules exprimant le FcγRIIIa. Dans ce cas, les plaques seront plutôt obtenues en les sensibilisant avec une concentration d'environ 0.01 à 1 μg/ml d'anticorps, pendant le même temps qu'indiqué plus haut.

Afin de permettre l'activation des cellules exprimant le FcγRIIIa, l'incubation de ces cellules avec les anticorps monoclonaux doit de préférence durer au moins 1h de préférence 2 à 4 heures, avant l'analyse des différents marqueurs de la réponse.

Dans une mise en oeuvre particulière de la méthode selon l'invention, une étape (ii-bis) de stimulation des cellules exprimant le FcγRIIIa, par un mélange d'un ester de phorbol (par exemple, le Phorbol 12-myristate 13-acetate (PMA)) et d'un ionophore calcique (Cal) (par exemple, le calcium ionophore A23187), est ajoutée à titre de contrôle positif de la capacité de réponse (globale) desdites cellules. Cette étape est particulièrement utile lorsque la méthode est utilisée pour effectuer un diagnostic prédictif de la réponse à un traitement par des anticorps monoclonaux, pour un patient à un stade avancé d'une maladie impliquant le système immunitaire (telle que l'infection par le VIH).

A l'issue de l'incubation, la méthode de l'invention peut avantageusement comporter la séquence suivante :

(iv) les cellules exprimant le FcγRIIIa sont marquées avec un anticorps anti-CD16 couplé à un marqueur,

(v) les cellules exprimant le FcγRIIIa sont perméabilisées, puis marquées avec un anticorps monoclonal anti-IFNγ couplé à un marqueur, et/ou avec un anticorps monoclonal anti-TNFα couplé à un marqueur,

(vi) les cellules ainsi marquées sont analysées par cytométrie en flux.

Selon une variante destinée à affiner les informations recueillies sur l'activation des cellules NK par un anticorps monoclonal, la méthode décrite ci-dessus peut être modifiée en co-stimulant les cellules NK, aux étapes (i) et (ii), avec des anticorps dirigés contre un ou plusieurs des récepteurs membranaires choisis dans la liste non exhaustive suivante : NKp30, NKp46, NKG2D, DNAM (CD226), CD2, CD27, 2B4 (CD244), CD11a, CD11b, CD45 et CD160 (en plus de la stimulation par l'anticorps monoclonal à tester, d'une part, et par l'anticorps anti-FcγRIIIa utilisé à titre de contrôle, d'autre part). Cette co-stimulation peut être effectuée en adsorbant, sur le même support que les anticorps à tester (ou contrôles), des anticorps monoclonaux dirigés contre le ou les récepteur(s) en question.

Suivant le but dans lequel elle est mise en oeuvre, la méthode de l'invention peut utiliser différentes sources et niveaux de purification de cellules exprimant le FcγRIIIa. Par exemple, il peut s'agir d'une lignée cellulaire (NK, ou lymphocytes T), ou de cellules NK ou de lymphocytes T isolés à partir d'un ou plusieurs prélèvements sanguins (par exemple, des cellules NK purifiées en utilisant un protocole tel que décrit dans la partie expérimentale ci-dessous). La méthode ci-dessus peut également être appliquée à des monocytes, mais avec un intérêt moindre. Il peut également s'agir de cellules exprimant le FcγRIIIa après transfection du gène *FCGR3A* (modifié ou non) et en association ou non avec le gène *FCERIG* codant la molécule transductrice FcR-γ. Lorsque l'invention est mise en oeuvre sur des cellules provenant de prélèvements sanguins, ces cellules peuvent être soit purifiées, soit présentes dans des fractions comportant plusieurs types cellulaires, telles que les cellules mononucléées du sang périphérique *(peripheral blood mononuclear cells ;* PBMC) ou les lymphocytes du sang périphérique *(peripheral blood lymphocytes ;* PBL) ; de façon avantageuse, la méthode de l'invention peut également être mise en oeuvre sur un échantillon de sang total, provenant de donneurs sains ou de malades. Lorsque les cellules utilisées n'ont pas été complètement purifiées, par exemple lorsque la méthode est mise en oeuvre sur des échantillons de sang total, les cellules sont de préférence également marquées avec un anticorps monoclonal anti-CD56 couplé à un marqueur et, le cas échéant, avec un anticorps monoclonal anti-CD3 et/ou un anticorps monoclonal anti-CD14 couplé à un autre marqueur, avant l'étape d'analyse par cytométrie en flux. Ce marquage, effectué de préférence avant la perméabilisation des cellules, permet de distinguer les différents types cellulaires de l'échantillon (cellules NK CD56+CD3-, lymphocytes T CD3+, monocytes CD14+, ...).

Une première application de la méthode décrite ci-dessus dans ses différentes variantes est d'effectuer un test diagnostic prédictif de la réponse d'un individu à un traitement par un anticorps monoclonal thérapeutique. Dans ce cas, la méthode est mise en oeuvre avec, bien entendu, l'anticorps (ou les anticorps) qu'on envisage d'administrer au patient, et des cellules exprimant le FcγRIIIa provenant du patient. Ces cellules peuvent être purifiées (par exemple, des cellules NK isolées à partir d'un échantillon sanguin), ou non (la méthode est alors mise en oeuvre sur un échantillon de sang total du patient).

La méthode selon l'invention peut également être utilisée pour quantifier les fonctions effectrices des cellules exprimant le FcγRIIIa en réponse aux anticorps monoclonaux, ou pour comparer la capacité de différents anticorps monoclonaux recombinants à induire la diminution d'expression du FcγRIIIa la cytotoxicité dépendante des anticorps (ADCC) et la production de cytokines dépendante des anticorps (ADCP) par des cellules exprimant le FcγRIIIa, notamment des cellules NK.

Comme mentionné plus haut, les méthodes de l'invention constituent un outil de recherche performant pour améliorer l'efficacité des anticorps monoclonaux recombinants à visée thérapeutique, par exemple pour tester les effets de modifications de leur portion Fc et/ou l'effet de différents adjuvants tels que l'IL-2 ou l'IL-12.

La présente invention fournit également une méthode alternative à celle utilisée actuellement par la Pharmacopée européenne pour effectuer un contrôle qualité des fontions de la portion Fc des immunoglobulines (Ig) administrées par voie intra-veineuse (IVIg). La technologie employée actuellement pour évaluer ces fonctions est en effet fondée sur l'activation du complément et non sur le recrutement des FcR, ce qui limite l'intérêt de son éventuelle utilisation dans le secteur des AcMor. Il existe aujourd'hui un besoin d'une technologie de référence permettant d'évaluer les fonctions effectrices des AcMor dépendantes des FcR, et particulièrement vis-à-vis d'un FcR dont l'implication clinique est démontrée, ce qui est incontestablement le cas du FcγRIIIa, et indépendante de l'antigène visé. La méthode de l'invention peut donc être avantageusement utilisée pour cela.

La présente invention porte également sur une trousse utilisable pour la mise en oeuvre de la méthode selon la présente invention, comprenant au moins les éléments suivants :

- un anticorps monoclonal anti-CD16 couplé à un marqueur,

- un anticorps monoclonal anti-IFNγ couplé à un marqueur et/ou un anticorps monoclonal anti-TNFα couplé à un marqueur, et
- une plaque comportant au moins un puits recouvert d'un anticorps monoclonal dirigé contre le récepteur FcγRIIIa.

L'homme du métier saura compléter cette trousse pour permettre la mise en oeuvre des différentes variantes de la méthode, décrites ci-dessus.

La présente invention est illustrée par les figures et la description expérimentale qui suit.

## LEGENDES DES FIGURES

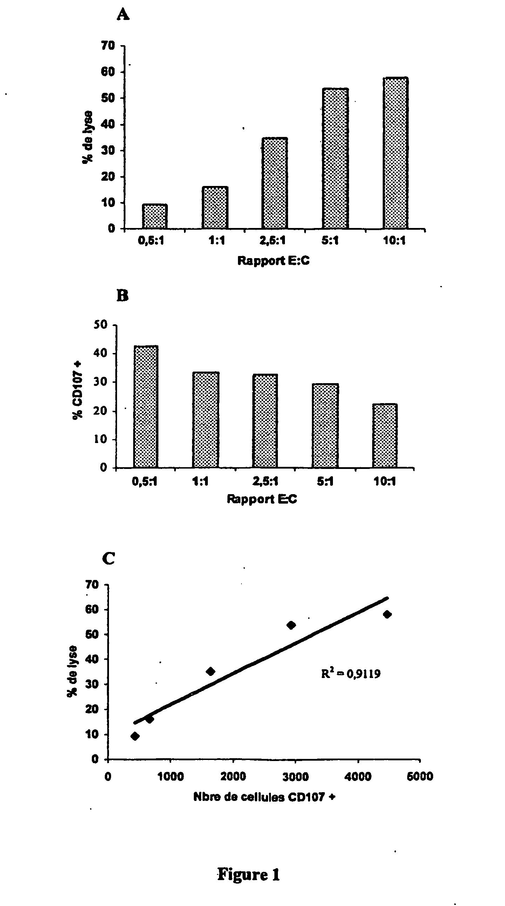

**Figure 1 : ADCC et dégranulation des cellules NK après incubation avec des cellules Daudi sensibilisées par le rituximab.** Les cellules NK purifiées ont été incubées avec des cellules Daudi marquées par du $^{51}$Cr (A) ou avec **des** cellules Daudi non marquées en présence d'un AcMo anti-CD107-PC5 (B) aux rapport effecteurs sur cibles (E : C) indiqués en présence de 2μg/ml de rituximab, pendant 4h à 37°C . **A)** La radioactivité a été mesurée dans les surnageants et les pourcentages de lyse ont été calculés selon la formule indiquée dans les matériels et méthodes. **B)** L'évaluation du pourcentage de cellules NK CD107$^+$ a été faite par cytométrie en flux, après marquage par un AcMo anti-CD56-PE (B). **C)** Le pourcentage de lyse a été exprimé par rapport au nombre total de cellules NK CD 107$^+$ (% de cellules CD56+CD107+ x nombre de cellules NK incubées au rapport E : C considéré).

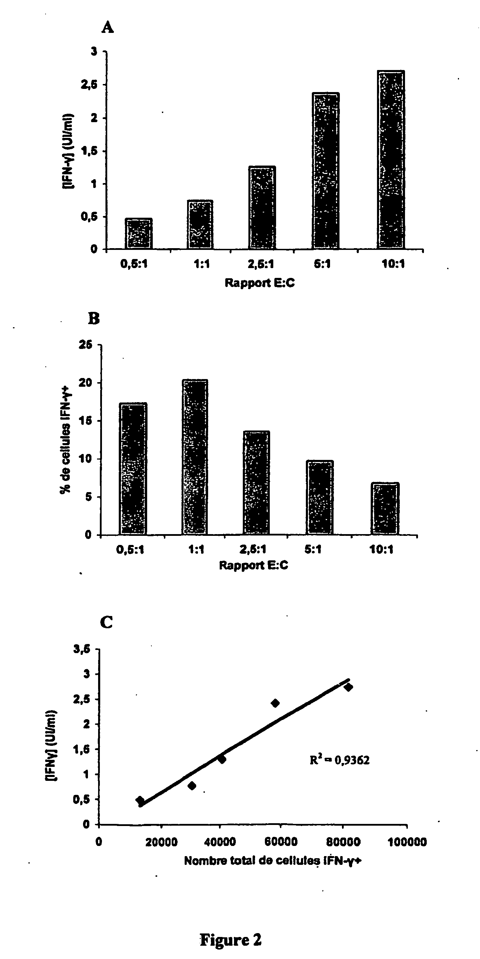

**Figure 2 : ADCP et synthèse d'IFNγ par les cellules NK après incubation avec des cellules Daudi sensibilisées par le rituximab.** Les cellules NK purifiées ont été incubées avec des cellules Daudi aux rapport E : C, indiqués en présence de 2 μg/ml de rituximab et en absence (A) ou en présence (B) de brefeldine A utilisée pour bloquer la sécrétion pendant 4h à 37°C. **A)** La concentration d'IFNγ dans le surnageant a été mesurée par méthode ELISA. **B)** L'évaluation du pourcentage de cellules NK IFNγ+ a été faite par cytométrie en flux, après marquage par un AcMo anti-CD56PC5, puis perméabilisation et marquage avec un AcMo anti IFNγ-PE. C) La concentration d'IFNγ dans le surnageant a été exprimée en fonction du nombre total de cellules NK IFNγ+ (% de cellules CD56+ IFNγ + x nombre de cellules NK incubées au rapport E : C considéré).

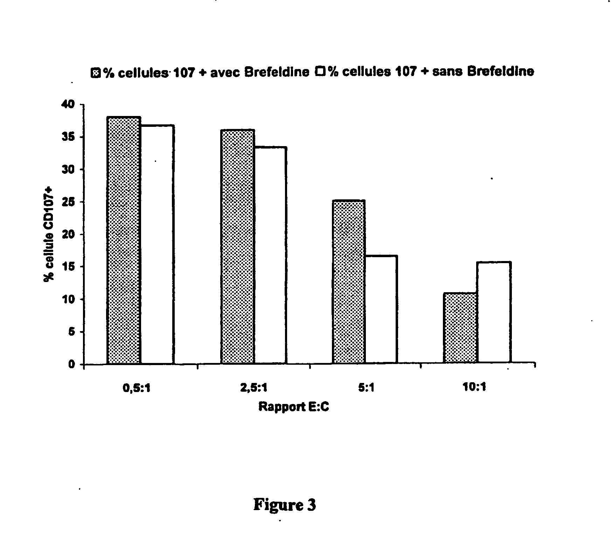

**Figure 3: Influence de la brefeldine A sur la dégranulation des cellules NK**. Les cellules NK purifiées ont été incubées avec des cellules Daudi en présence d'un AcMo anti-CD107- PC5 aux rapport E : C indiqués, en présence de 2μg/ml de rituximab et en absence (barres blanches) ou en présence (barres avec des points) de brefeldine A utilisée pour bloquer la sécrétion pendant 4h à 37°C. L'évaluation du pourcentage de cellules NK CD107+ a été faite par cytométrie en flux, après marquage par un AcMo anti-CD56-PE.

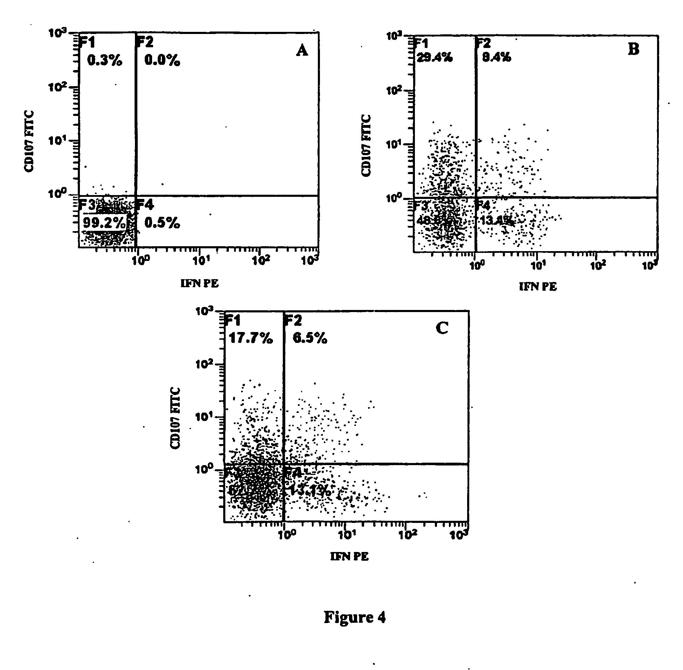

**Figure 4 : Dégranulation et synthèse d'IFNγ par les cellules NK après incubation avec des cellules Daudi sensibilisées par le rituximab ou l'AcMo anti-CD16 3G8.** Les cellules NK purifiées ont été incubées avec des cellules Daudi en présence d'un AcMo anti-CD107- PC5 au rapport E : C de 0,5 : 1, en présence de brefeldine A et en absence **(A)** ou en présence de 2 μg/ml de rituximab **(B)** ou de 2 μg/ml de l'AcMo anti-CD16 3G8 **(C)** pendant 4h à 37°C. Les cellules ont ensuite été marquées avec un AcMo anti-CD56, puis perméabilisées,marquées avec un AcMo anti IFNγ-PE et analysées par cytométrie en flux. Les cytogrammes représentent l'expression du CD107 (en ordonnées) et de l'IFNγ intracellulaire (en abscisses) des cellules CD56+.

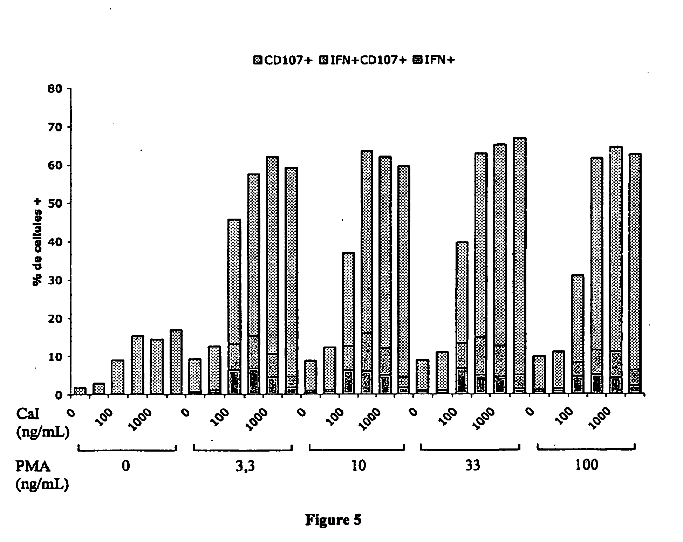

**Figure 5 : Dégranulation et synthèse d'IFNγ par les cellules NK stimulées par le PMA et le Cal**. Les cellules NK purifiées ont été incubées avec les concentrations indiquées de PMA et/ou de Cal en présence d'un AcMo anti-CD107-PC5 et en présence de Brefeldine A pendant 4h à 37°C. Les cellules ont ensuite été perméabilisées et marquées avec un AcMo anti IFNγ-PE et analysées par cytométrie en flux. Pour chaque condition de stimulation, les pourcentages des cellules NK CD 107-IFNγ+ sont représentés en gris, ceux des cellules NK CD107+IFNγ- sont représentés en blanc avec des points et ceux des cellules doublement positives (CD107+ IFNγ+) en gris avec des points.

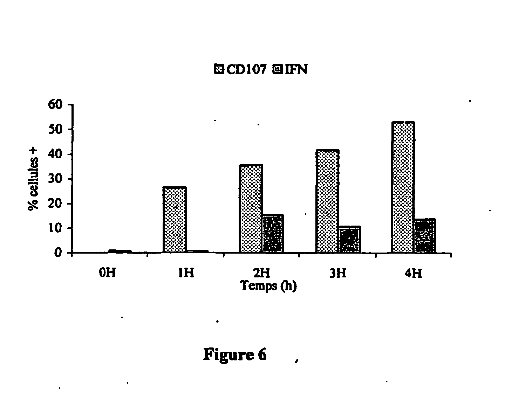

**Figure 6 : Cinétique de la dégranulation et de la synthèse d'IFNγ par les cellules NK stimulées par le PMA et le Cal**. Les cellules NK purifiées ont été traitées comme cela est décrit dans la légende de la figure 5, à l'exception du temps d'incubation, qui a été compris entre 1h et 4h. Les pourcentages de cellules NK IFNγ+ sont représentés en gris et ceux des cellules NK CD107+ sont représentés en blanc avec des points.

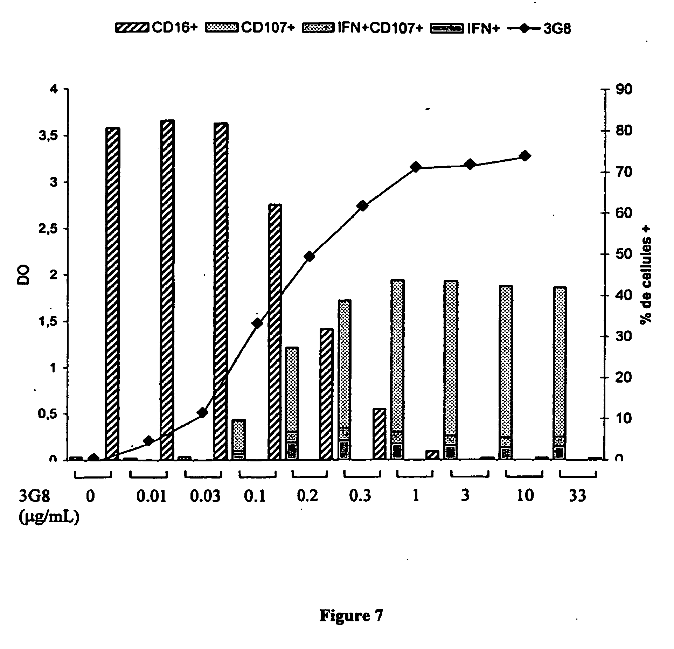

**Figure 7 : Expression du CD16, dégranulation et synthèse d'IFNγ par les cellules NK après incubation dans des plaques sensibilisées par l'AcMo anti-CD16 3G8.** Les plaques ont été sensibilisés pendant 12h avec les concentrations indiquées de l'AcMo anti-CD16 3G8 dont la fixation a été révélée par une technique ELISA en utilisant un Ac anti-IgG murine couplé à la peroxydase. La courbe représente les absorbances (en ordonnées à gauche) en fonction de la concentration de 3G8 utilisée pour la sensibilisation de la plaque (en abscisse). Les cellules NK purifiées ont été incubées dans des plaques sensibilisées avec l'AcMo anti-CD16 3G8 en présence d'un AcMo anti-CD107-PC5 et en présence de brefeldine A pendant 4h à 37°C. Les cellules ont ensuite été marquées par un AcMo anti-CD16-FITC, perméabilisées et marquées avec un AcMo anti IFNγ-PE et analysées par cytométrie en flux. Les pourcentages de cellules positives (en ordonnées à droite) sont représentés en fonction de la concentration sensibilisante de 3G8. Les pourcentages des cellule NK CD16+ sont en hachuré. Ceux des cellules NK CD107+IFNγ-, CD107-IFNγ+ et CD107+IFNγ+ sont représentés comme dans la figure 5.

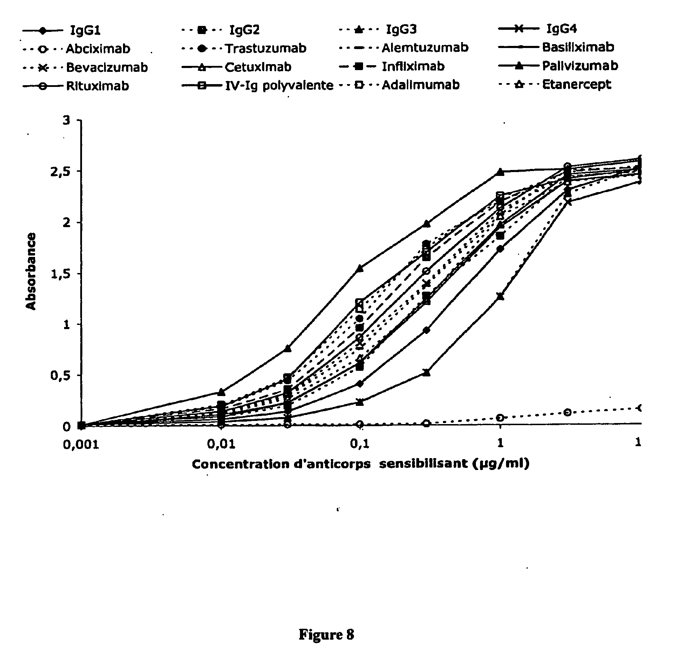

**Figure 8 : Adsorption des AcMor thérapeutiques et des IgG humaines sur les plaques**. Les plaques ont été sensibilisées avec les concentrations indiquées de chaque AcMor ou de chaques sous-classe d'IgG humaine pendant une nuit à 4°C. La quantité d'AcMor ou d'IgG adsorbée a été déterminée par un test ELISA à l'aide d'un anticorps anti-Fc couplé à la peroxydase. Les valeurs d'absorbance représentées correspondent à la différence des absorbances mesurées à 492 et 620 nm.

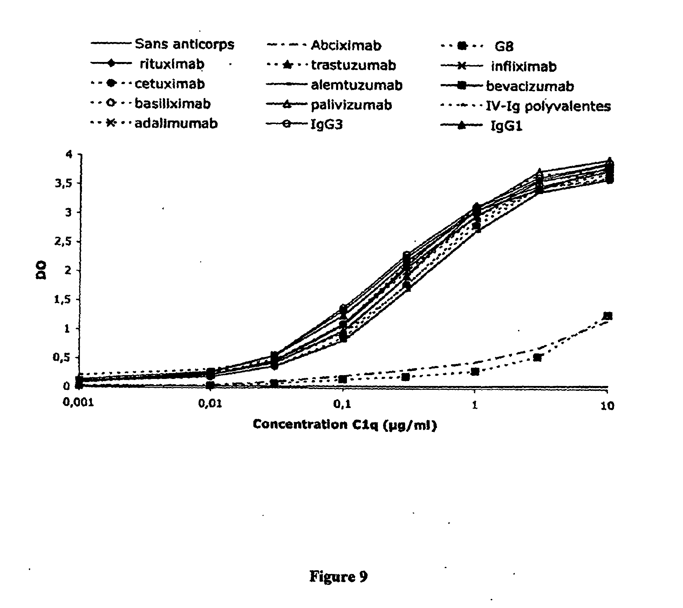

**Figure 9 : Fixation du C1q sur les AcMor thérapeutiques et sur les IgG humaines adsorbées sur les plaques**. Les plaques ont été sensibilisées avec 5 μg/mL de chacun des AcMor ou d'etanercept ou d'IgG polyvalente ou d'IgG1 humaine ou d'IgG3 humaine ou de l'AcMo anti-CD16 3G8 pendant une nuit à 4°C. Les plaques ont ensuite été incubées avec les concentrations indiquées de C1q humain. La quantité de C1q fixée sur les anticorps a ensuite été mesurée à l'aide d'un anticorps anti-C1q couplé à la peroxydase. Les valeurs d'absorbance représentées correspondent à la différence des absorbances mesurées à 492 et 620 nm.

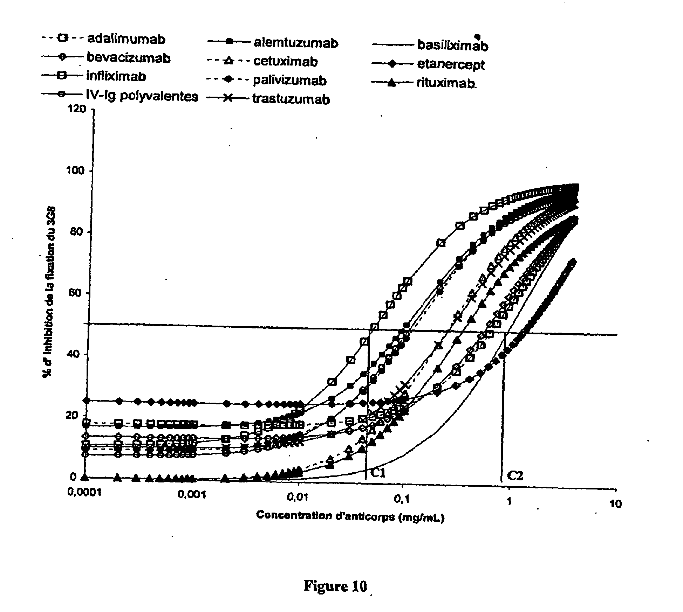

**Figure 10 : Affinité comparée des AcMor pour le FcγRIIIa des cellules NK**. Les cellules NK d'un donneur (de génotype 158-FF) ont été purifiées, et incubées avec les concentrations indiquées de chacun des AcMor ou d'IgG polyvalentes puis avec une concentration suboptimale (0,1μg/mL) de l'AcMo anti-CD16 3G8 conjugué au FITC. Le pourcentage d'inhibition de la fixation du 3G8-FITC (ordonnée) pour chacun des produits testés a été calculé d'après la formule décrite dans la partie "matériels et méthodes".

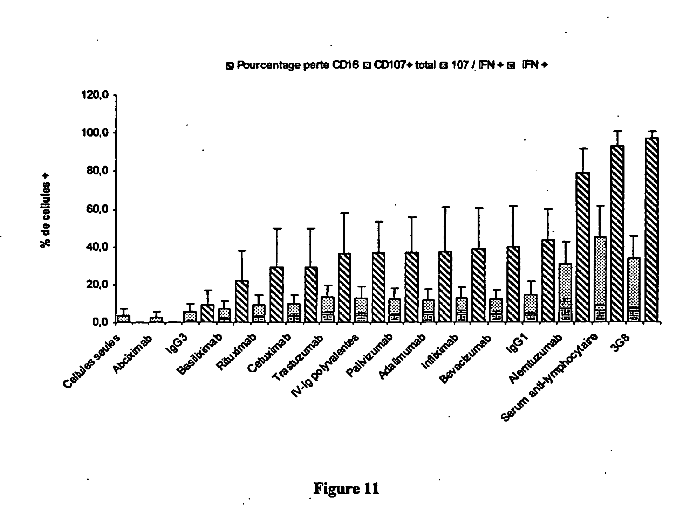

**Figure 11 : Perte d'expression du CD16, dégranulation et synthèse d'IFNγ par les cellules NK après incubation dans des plaques sensibilisées : comparaison des réponses moyennes induites par les AcMor thérapeutiques, les IgG humaines ou l'AcMo anti-CD16 3G8**. Les plaques ont été sensibilisés avec 5 μg/mL de chacun des AcMor ou d'etanercept ou d'IgG polyvalente ou d'IgG1 humaine ou d'IgG3 humaine ou de sérum anti-lymphocytaire ou de l'AcMo anti-CD16 3G8 pendant une nuit à 4°C. Les cellules NK purifiées ont été incubées et analysées comme cela est décrit dans la figure 7. Les pourcentages des cellules NK ayant perdu l'expression du CD16 sont en hachuré. Ceux des cellules NK CD107-IFNγ+, CD107+IFNγ- et CD107+IFNγ+ sont représentés comme dans la figure 5. Les résultats sont exprimés en moyenne $\pm$ écart type et ont été obtenus au cours de 8 expériences.

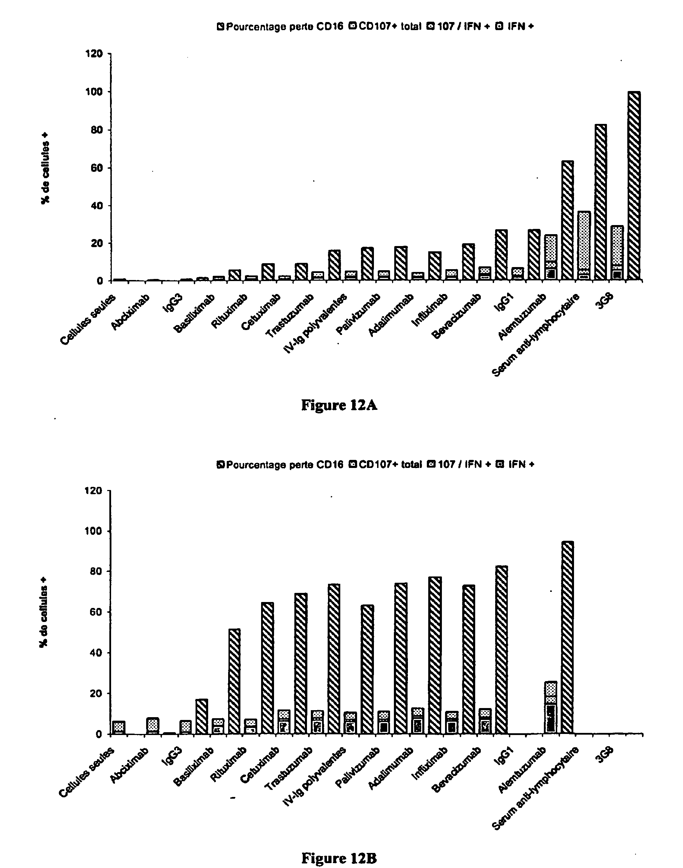

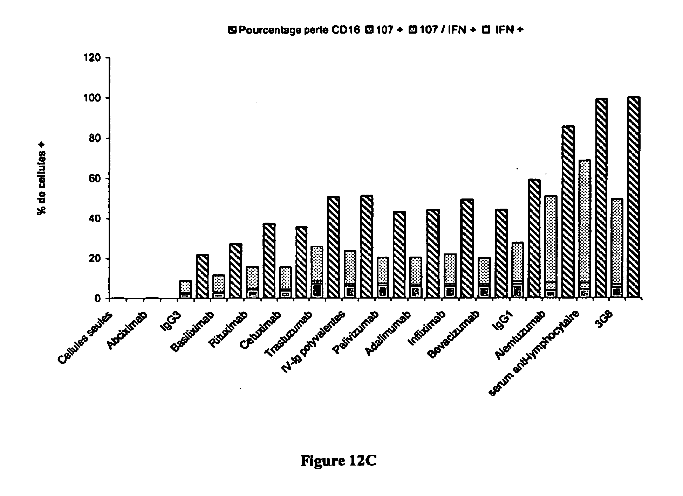

**Figure 12 : Perte d'expression du CD16, dégranulation et synthèse d'IFN-γ par les cellules NK après incubation dans des plaques sensibilisées : comparaison des réponses des cellules NK de 3 donneurs induites par les AcMor thérapeutiques, les IgG humaines ou l'AcMo anti-CD16 3G8.** Les résultats individuels obtenus avec les cellules NK de 3 des donneurs (A, B et C) de la figure 11 sont figurés.

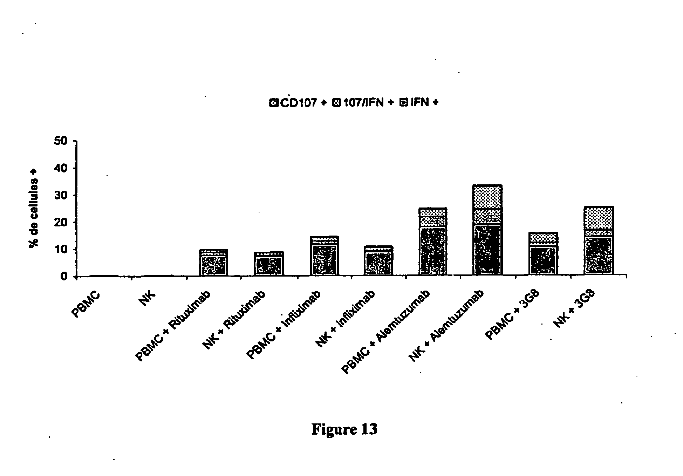

**Figure 13 : Dégranulation et synthèse d'IFN-γ par les cellules NK purifiées ou par les PBMC CD56+ après incubation dans des plaques sensibilisées par 3 AcMor thérapeutiques, ou l'AcMo anti-CD16 3G8**. Les plaques ont été sensibilisées ou non avec 5 μg/mL de chacun des AcMor thérapeutiques ou de l'AcMo anti-CD16 3G8 pendant une nuit à 4°C. Les PBMC ou les cellules NK purifiées d'un même donneur ont été incubées dans les plaques sensibilisées en présence d'un AcMo anti-CD107-PC5 et en présence de brefeldine A pendant 4h à 37°C. Les cellules ont ensuite été marquées par un AcMo anti-CD56-FITC, perméabilisées et marquées avec un AcMo anti IFNγ-PE et analysées par cytométrie en flux. Les pourcentages des cellules NK CD107+IFNγ+-, CD107+IFNγ- et CD107+IFNγ+ sont représentés comme dans la figure 5.

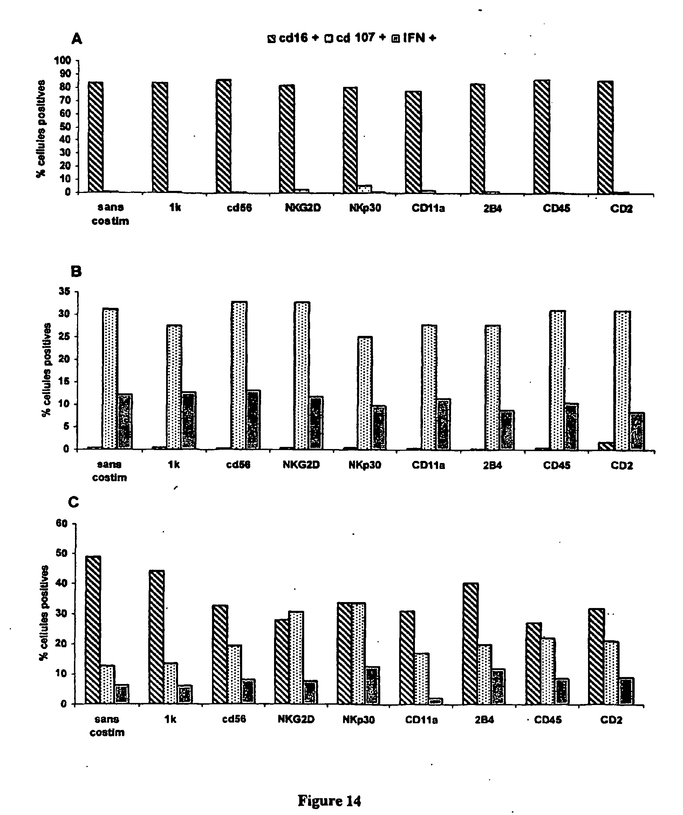

**Figure 14 : Expression de CD16, dégranulation et synthèse d'IFNγ par les cellules NK après incubation dans des plaques co-sensibilisées par l'AcMo anti-CD16 3G8 ou l'infliximab et par des AcMo dirigés contre les molécules co-activatrices des cellules NK**. Les plaques ont été sensibilisées pendant 12 h avec ou sans 1,5 μg/mL final d'AcMo anti-CD16 3G8 ou d'infliximab, en association ou non avec 0,5 ou 5μg/mL final d'IgG1 kappa murine (IgG1k) utilisée comme témoin négatif ou d'un des AcMo indiqués qui sont dirigés contre les molécule activatrice ou co-activatrices des cellules NK. Les cellules NK purifiées ont été incubées et analysées comme cela est décrit dans la figure 7. Les histogrammes hachurés, pointillés et grisés représentent respectivement les pourcentages des cellules CD16+, CD107+ et IFN-γ+. obtenus après incubation (A) dans des plaques sensibilisées respectivement par les seuls AcMo dirigés contre les molécules activatrices ou co-activatrices, (B) par ces AcMo et par l'AcMo anti-CD16 3G8 et (C) par ces AcMo et par l'infliximab.

## EXEMPLES

Les exemples expérimentaux qui suivent ont été réalisés en utilisant les matériels et méthodes décrits ci-après.

*Anticorps et activateurs chimiques*

Le basiliximab (Simulect®, Novartis Pharma), le cetuximab (Erbitux®, Merck Lipha sante), l'infliximab (Remicade®, Schering-plough), le rituximab (Mabthéra®, Rituxan®, Roche) et l'abciximab (Réopro®, Lilly France) sont des AcMor thérapeutiques chimériques, dirigés respectivement contre le CD25, l'EGFR *(epidermal growth factor receptor),* le TNFα, le CD20 et l'intégrine GPIIb/IIIa. Il faut cependant noter que l'abciximab ne contient pas de portion Fc. L'alemtuzumab (Mabcampath®, Schering SA), le bevacizumab (Avastin®, Roche), le palivizumab (Synagis®, Abbott France), le trastuzumab (Herceptin®, Roche) sont des AcMor humanisés dirigés respectivement contre le CD52, le VEGF *(vascular endothelial growth factor),* la protéine de fusion du virus respiratoire syncytial, et l'antigène ErbB2. L'adalimumab (Humira®, Abbott France), est un AcMor totalement humain dirigé contre le TNFα. L'etanercept (Enbrel®, Wyeth-Lederlé) est un récepteur du TNFα fusionné à une portion Fc d'IgG humaine. Les IVIg (Tegeline®, LFB) sont des immunoglobulines humaines polyvalentes préparées à partir de plasmas humains et le serum anti-lymphocytaire (Thymoglobuline®, Genzyme) est un serum de lapin dirigé contre les antigènes des thymocytes humains. L'AcMo anti-CD16 clone 3G8, l'Ac anti-CD16 conjugué à l'isothiocyanate de fluorescéine (CD16-FITC) clone 3G8, l'AcMo anti-IFNγ conjugué à la phycoerythrine (IFNγ-PE) clone 45.15, l'AcMo anti-CD56 conjugué à la phycoerythrocyanine 5 (CD56-PC5) et à la phycoerythrine (CD56-PE), les contrôles isotypique-PE, -PC5 et -FITC proviennent tous de Beckman Coulter (Villepinte, France). Ce sont tous des IgG1 murines. L'AcMo anti-CD107 conjugué à la phycoerythrocyanine 5 (CD107-PC5) et le contrôle isotypique-PC5 proviennent de BD Biosciences (Le Pont de Claix, France).

Le Phorbol 12-myristate 13-acetate (PMA), et le Calcium Ionophore (CaI) A23187 ont été fournis par Sigma (L'Isle d'Abeau Chesne, France).

*Culture des cellules cibles*

La lignée cellulaire cible utilisée est la lignée lymphoblastoïde Daudi. Elle est dérivée d'un lymphome de Burkitt. Elle est résistante à la cytotoxicité naturelle par les NK (Dall'Ozzo et al., 2004) et exprime à sa surface l'antigène CD20 humain. Les cellules Daudi ont été cultivées dans une étuve à 37°C sous 5% de $CO_2$ en atmosphère humide. Le milieu de culture était constitué de RPMI (Eurobio, Les Ulis, France) contenant de la L-Glutamine (Bio Whittaker Europe), 1 mM de pyruvate de sodium (Invitrogen), de 50 UI/ml de pénicilline et de 50 μg/ml de streptomycine (Bio Whittaker Europe). Deux fois par semaine, au moment du changement de milieu, ce mélange a été complété avec 10% de sérum de veau foetal (SVF) (Invitrogen) décomplémenté à 56°C pendant 30 min. Les cellules ont été cultivées dans des flasques de 75 $cm^2$ de surface (BD Biosciences), sous un volume moyen de 30 ml. La viabilité des cellules a été régulièrement vérifiée par le test d'exclusion au Bleu Trypan.

*Préparation des cellules NK*

Les prélèvements de sang veineux périphérique (40 à 60 ml) ont été réalisés chez des donneurs sains après consentement écrit, dans des tubes de 6 ml avec anticoagulant (héparinate de Sodium). Le sang était dilué au 2/3 dans du milieu RPMI 1640 (Eurobio), et 7 ml était déposés sur 4 ml de solution de séparation lymphocytaire Lymphoprep® (Eurobio). Après une centrifugation de 20 min à 700 g, les cellules mononuclées (PBMC : *peripheral blood mononuclear cells)* formaient à l'interface Lymphoprep®/plasma un anneau blanchâtre, récupéré puis lavé avec 10 ml de milieu RPMI 1640. Après centrifugation de 10 min à 560 g, le culot cellulaire obtenu était repris dans 1ml de tampon PBS contenant 2 mM d'EDTA (Sigma) et du SVF à 10% final : tampon PES (PBS/SVF/EDTA). Une numération à la cellule de Mallassez était réalisée sur un échantillon de 5 μL de suspension cellulaire dans 295 μl de Bleu Trypan, au microscope objectif x40.

L'isolement des cellules NK à partir des PBMC a été réalisé avec le kit d'immuno-sélection magnétique négative et indirecte (NK cell Isolation Kit II MACS®) de Miltenyi Biotec (Paris, France), selon les instructions du fabricant : la suspension de PBMC était centrifugée 10 min à 560 g, puis reprise dans du tampon PES, à raison de 40 μl pour $10^7$ PBMC. Après ajout de 10 μl de solution A, contenant un mélange d'AcMo anti-CD3, anti-CD4, anti-CD14, anti-CD15, anti-CD 19, anti-CD36, anti-CD123, et anti-CD235a (Glycophorine A) couplés à la biotine pour $10^7$ PBMC, une incubation de 10 min à 4°C était réalisée. 30 μl de tampon PES et 20 μl de solution B contenant des billes couplées à un Ac anti-biotine étaient ensuite ajoutés pour $10^7$ PBMC. Une incubation de 15 min à 4°C était ensuite réalisée, suivi d'un lavage dans du tampon PES et d'une centrifugation de 10 min à 560 g. Le culot était repris dans 500 μl de tampon PES et déposé sur une colonne de séparation magnétique type LS VARIO MACS® (Miltenyi Biotec) placée sur un aimant quadroMACS® (Miltenyi Biotec). Les cellules NK étaient ainsi récupérées dans la solution éluée, et subissaient un lavage avec 5 mL de tampon PES. Après centrifugation 10 min à 560g, ces cellules étaient reprises dans 250 à 1000 μl de tampon PES, en fonction du nombre de PBMC initial. Une numération à la cellule de Mallassez était réalisée sur un échantillon de 5μl de suspension cellulaire dans 295 μl de Bleu Trypan, au microscope, à l'objectif x40.

*Stimulation des cellules NK*

*Sensibilisation des plaques de culture par les AcMor thérapeutiques, les IgG humaines; le serum anti-lymplocytaire et l'AcMo anti-CD16.*

Les plaques NUNC Maxisorp® de 96 puits (Fischer Labosi, Elancourt, France) ont été sensibilisées pendant 12 heures à 4°C par des concentrations comprises entre 0,01 $\mu$g/ml et 10 $\mu$g/ml de chacun des AcMor thérapeutiques ou d'etanercept ou d'IgG1 humaine ou d'IgG3 humaine ou d'IgG humaine polyvalente ou de serum anti-lymphocytaire ou de l'AcMo anti-CD16 3G8, sous un volume de 200 $\mu$l.

La plaque a ensuite été lavée 3 fois avec du PBS TWEEN (45 $\mu$l de TWEEN 20 (Sigma) dans 100 ml de PBS), puis saturée 30 min avec de l'albumine sérique bovine à 1% (BSA)(Sigma), et de nouveau lavée 3 fois avec du PBS TWEEN avant utilisation. Dans les expériences de costimulation, les plaques ont été sensibilisées pendant 12 heures à 4°C par 100 $\mu$L d'une solution d'AcMo anti-CD16 clone 3G8 (Beckman Coulter) ou d'infiximab à 3 $\mu$g/mL préalablement mélangés ou non avec 100 $\mu$l d'une solution d'AcMo à 10 ou à 1 $\mu$g/ml : IgG1 murine clone MOPC-31C comme témoin, AcMo anti-NKG2D clone 1D11, AcMo anti-CD11 a clone G43-25B, AcMo anti-2B4 clone 2-69, AcMo anti-CD45 clone HI30 (provenant de BD Biosciences), AcMo anti-CD2 clone 6F10.3 AcMo anti-NKp30 clone Z25, AcMo anti-CD56 clone C218 (provenant de Beckman Coulter).

*Stimulation des cellules NKpar l'AcMo anti-CD16 immobilisé*

Les cellules NK ont été déposées dans les plaques sensibilisées à raison de $10^5$ cellules/puits sous un volume final de 200 $\mu$l et incubées en présence de 5$\mu$l d'AcMo anti-CD107-PECy5 ou -PE et d'un bloqueur de sécrétion protéique : le BD GolgiPlug® contenant la Brefeldine A (BD Biosciences®), à raison de 1 $\mu$l/ml de solution cellulaire, pendant 4 heures à 37°C, sous 5% de $CO_2$ en atmosphère humide.

*Stimulation des cellules NK par les cellules Daudi en présence de rituximab ou de 3G8.*

Les cellules Daudi ($2x10^4$) ont été déposées dans des plaques de 96 puits à fond plat (Falcon 3047, BD Biosciences) avec de $10^4$ à $2x10^5$ cellules NK (rapport effecteurs sur cibles E : C compris entre 0,5 : 1 et 10 :1) en présence de 2 $\mu$g/ml de rituximab ou de 3G8, 5 $\mu$l de l'AcMo anti-CD107-PC5 et en absence ou en présence de BD GolgiPlug®, à raison de 1 $\mu$l/ml de solution cellulaire, sous un volume total de 200 $\mu$l pendant 4 heures à 37°C, sous 5% de $CO_2$ en atmosphère humide.

*Stimulation des cellules NK par les cellules Daudi en présence de rituximab pour le dosage de l'IFN$\gamma$ sécrété*

Les cellules Daudi ($2x10^4$) ont été déposées dans des plaques de 96 puits à fond plat comme décrit au paragraphe précédent, en présence de 2 $\mu$g/ml de rituximab sous un volume total de 200 $\mu$l pendant 4 heures à 37°C, sous 5% de $CO_2$ en atmosphère humide. 50 $\mu$l de surnageant ont ensuite été prélevés puis congelés à -80°C en vue du dosage.

*Stimulation des cellules NK par le PMA et le CaI*

Les cellules NK ont été déposées dans les plaques à raison de $10^5$ cellules/puits sous un volume final de 200 $\mu$l et incubées en présence de 3,3 $\mu$g/ml à 100 $\mu$g/ml PMA et/ou de 33 $\mu$g/ml à 3300 $\mu$g/ml de CaI, en présence de 5$\mu$l d'AcMo anti-CD107-PECy5 et de BD GolgiPlug® à raison de 1 $\mu$l/ml de solution cellulaire, pendant 4 heures à 37°C, sous 5% de $CO_2$ en atmosphère humide. Pour l'étude cinétique, les temps d'incubation ont été compris entre 1h et 4h.

*Détection de l'IFN$\gamma$ intracellulaire*

La détection de l'IFN$\gamma$ a été réalisée d'après les techniques publiées (Mendes et al., 2000; Vitale et al., 2000) : après incubation, les cellules NK ont été transférées dans des tubes de cytométrie (Beckman Coulter). Les cellules ont été lavées avec 2 ml de tampon PBS, centrifugées 5 min à 560 g, et incubées ou non pendant 30 min à 4°C avec 5 $\mu$l de l'AcMo anti CD16-FITC. Les cellules ont ensuite été lavées avec 2 ml de PBS et de nouveau centrifugées 5 min. à 4°C et incubées pendant 20 min. à 4°C avec 270 $\mu$l d'une solution BD cytofix/cytoperm kit® (BD Biosciences). Après un lavage avec 2 ml d'une solution de lavage Perm/Wash® (BD Biosciences), les cellules ont été centrifugées 5 min à 4°C, puis incubées 30 min à 4°C avec 4 $\mu$l d'anticorps anti IFN$\gamma$-PE. Les cellules ont été de nouveau lavées avec 2 ml de la solution de lavage Perm/Wash®, et centrifugées 5 min. à 4°C à 560 g. Finalement le culot cellulaire a été repris dans 400$\mu$l de tampon PBS pour l'analyse des cellules par cytométrie en flux.

*Test de cytotoxicité*

*Préparation des cellules cibles*

$10^7$ cellules Daudi ont été reprises dans 300 $\mu$l de milieu RPMI 1640, et incubées avec 3 MBq (100$\mu$Ci) de $^{51}Cr_2O4Na_2$ (Dupont-NEN, Les Ulis, France) pendant 90 min. à 37°C sous agitation régulière. Les cellules ont ensuite été lavées 2 fois avec 10 ml de milieu RPMI 1640 pour supprimer la radioactivité résiduelle non fixée. Après une incubation de 60 min. à 37°C, et deux nouveaux lavages en milieu RPMI 1640, la suspension cellulaire a été ajustée à une concentration de $4.10^5$ cellules/ml. La suspension cellulaire a ensuite été repartie dans une plaque Microtest® de 96 puits fond plat (BD Biosciences), à raison de 50 $\mu$l ($2\times10^4$ cellules) par puits.

*Mesure de la cytotoxicité cellulaire*

50 $\mu$l de rituximab à 2 $\mu$g/ml final ont été ajoutés à $2\times10^4$ cellules Daudi marquées au $^{51}Cr$. Les cellules effectrices ont ensuite été distribuées sous 100 $\mu$l dans les puits d'intérêt à des rapports rapports E : C compris entre 0,5 : 1 et 10 : 1. Après 4 h d'incubation à 37°C sous $CO_2$, 50 $\mu$l de surnageant ont été récupérés et évaporés durant une nuit en étuve sèche. La radioactivité a été mesurée par un compteur de particules $\gamma$ (Top count®, Packard Rungis France). Le relargage spontané (cpm spont) a été obtenu en incubant des cellules cibles avec 150 $\mu$l de milieu de culture seul, et le relarguage maximal (cpm max) en incubant des cellules cibles avec 150 $\mu$l de milieu de culture contenant 1% de Triton X100 (Sigma). Le % de lyse spécifique a été donné par le calcul :

$$\text{\% de lyse spécifique} = (\text{cpm test} - \text{cpm spont}) / (\text{cpm max} - \text{cpm spont}).$$

*Dosage de l'IFN$\gamma$ sécrété :*

Le dosage de l'IFN$\gamma$ a été réalisé sur les surnageants avec un kit de dosage immunoenzymatique d'IFN$\gamma$ (Enzyme Immunoassay kit°', Immunotech), selon le mode opératoire fourni. C'est un dosage immunoenzymatique de type sandwich en deux étapes : capture de l'IFN$\gamma$ par un anticorps monoclonal anti-IFN$\gamma$ fixé dans les puits d'une plaque de microtitration, puis liaison d'un deuxième Ac anti IFN$\gamma$-biotinylé, révélé par un conjugué Streptavidine-Peroxydase. L'activité enzymatique a ensuite été déterminée par l'addition d'un substrat chromogène. L'intensité de coloration développée, lue à 450 nm, est proportionnelle à la concentration en IFN$\gamma$ présente dans l'échantillon ou le standard. Les résultats sont exprimés en UI /ml.

*Détection des AcMor ou de l'AcMo anti-CD16 immobilisés*

Les plaques NUNC sensibilisées par les AcMor ont été lavées, puis une dilution au 1/4000ème de F(ab')$_2$ de chèvre anti-IgG humaine spécifique de la chaîne $\gamma$ conjugué à la peroxydase (Sigma) a été déposée à raison de 100 $\mu$l par puits. Les plaques NUNC sensibilisées par l'AcMo anti-CD16 3G8 ont été lavées, puis une dilution au 1/5000ème d'anticorps de chèvre spécifique de la portion Fc des IgG de souris conjugué à la peroxydase (Jackson Immuno Research Laboratories, Villepinte France) a été déposée à raison de 100 $\mu$l par puits. Après une incubation d'1 h à température ambiante suivie de trois lavages avec une solution de PBS-TWEEN (45 $\mu$l de TWEEN 20 (Sigma) dans 100 ml de PBS) une solution d'O-phenylènediamine à 0,5 mg/ml finale (Sigma), à raison de 100 $\mu$l par puit, a été ajoutée. Après une incubation de 30 min à température ambiante, 50 $\mu$l d'une solution de $H_2SO_4$ 2N (solution STOP), a été déposée dans chaque puit. La lecture de la plaque a été réalisée sur un spectrophotomètre iEMS reader MF® (Labsystems, Cergy pontoise France) à 492 et 620 nm de longueur d'onde. Les valeurs d'absorbance obtenues correspondaient à la différence des absorbances obtenues entre 492 et 620 nm.

*Mesure de l'affinité des AcMor et des IgG humaines pour le Fc$\gamma$RIIIa des cellules NK*

Les cellules NK purifiées ont été ajustées à la concentration de $15.10^6$ cellules/ml dans du PBS. 10$\mu$l de cette solution ont été incubées avec 90 $\mu$l des concentrations indiquées des AcMor thérapeutiques ou d'etanercept ou d'IgG humaines polyvalentes en PBS dans des tubes pour cytomètre de 5 mL (Beckman Coulter) pendant 30 min à 4°C. Les cellules ont ensuite été incubées avec 5 $\mu$l d'Ac Mo anti-CD56 PC5 et 45 $\mu$l d'AcMo anti-CD16-FITC 3G8 à 0,334 $\mu$g/ml (soit une concentration finale de 0,1 $\mu$g/mL) pendant 30 min à 4°C. Après un lavage avec 2 ml de PBS, les cellules ont été reprises avec 450 $\mu$l de PBS et analysées par cytométrie en flux. Les résultats ont été exprimés en % d'inhibition de la fixation de l'AcMo anti-CD16 3G8 selon la formule suivante: % = (Xmean en absence d'AcMo-Xmean en présence d'AcMor) x 100 / (Xmean en absence d'Ac Mor).

*Fixation du C1q sur les AcMor thérapeutiques et sur les IgG humaines adsorbées*

Les plaques sensibilisées avec 5 $\mu$g/ml des différents AcMor ou d'IgG1 humaine ou d'IgG3 humaine ou d'IgG polyvalentes humaines ou de l'AcMor anti-CD16 3G8 ont été lavées puis incubées pendant 2h à température ambiante avec les concentrations indiquées de C1q humain (Sigma) dilué en PBS. Après 3 lavages avec une solution de PBS-TWEEN, les plaques ont été incubées avec un anticorps polyclonal de mouton anti-C1q humain couplé à la peroxydase (Biogenesis, distribué par Absys, Paris, France) dilué en PBS-BSA 1%. Après une incubation d'1 h à température ambiante suivie de trois lavages avec une solution de PBS-TWEEN, une solution d'O-phenylènedianine à 0,5 mg/ml finale, à raison de 100 $\mu$l par puits, a été ajoutée. Après une incubation de 15 min à température ambiante, 50 $\mu$l d'une solution de $H_2SO_4$ 2N, a été déposée dans chaque puits. La lecture de la plaque a été réalisée sur un spectrophotomètre *iEMS reader MF®* à 492 et 620 nm de longueur d'onde. Les valeurs d'absorbance obtenues correspondaient à la différence des absorbances obtenues entre 492 et 620 nm.

*Analyse par cytométrie en flux*

Les cellules ont été analysées avec un cytomètre XL (Beckman Coulter) en utilisant le logiciel d'acquisition et d'analyse EXPO 32®. Pour chaque détermination, au minimum 4000 cellules ont été analysées. L'analyse des fluorescences a été conditionnée par une fenêtre sur un diagramme bi-paramétrique taille/structure (FSC/SSC = *Forward Scatter Channel/Side Scatter Channel).*

**Exemple 1 : Mesure de l'ADCC exercée par les cellules NK sur les cellules Daudi en présence de rituximab par le test de libération du $^{51}$Cr et par l'expression membranaire du CD107**

Il a été montré précédemment que les cellules NK purifiées lysaient efficacement des cellules cibles CD20+ Daudi en présence de rituximab (anticorps chimérique anti-CD20) à la concentration de 2 $\mu$g/ml dans un test de cyto-toxicité au $^{51}$Cr de 4h (Dall'Ozzo et al., 2004). Il a été récemment montré que l'expression du CD107 sur les cellules cytotoxiques permettait d'évaluer la dégranulation de celles-ci et représentait une mesure alternative à la cytotoxicité (Alter et al., 2004; Betts et al., 2003; Betts et al., 2004). Afin de tester la validité du test de dégranulation pour évaluer l'ADCC, de $10^4$ à $2\times10^5$ cellules NK purifiées d'un même donneur ont été incubées pendant 4h à 37°C en présence de 2 $\mu$g/ml de rituximab, avec $2\times10^4$ cellules Daudi préalablement marquées ou non avec du [51]Cr. La cytotoxicité a été mesurée en comptant la radioactivité libérée dans le surnageant par les cellules Daudi préincubées avec le [51]Cr. La dégranulation a été évaluée par cytométrie en flux après incubation des effecteurs et des cibles non marquées durant les 4 h du test avec un AcMo anti-CD107 conjugué au PC5 puis marquage par un AcMo anti-CD56 conjugué au PE. Comme le montre la figure 1A et comme attendu, le pourcentage de lyse des cellules Daudi a augmenté avec le rapport E : C.

La figure 1B montre que seule une fraction (dans tous les cas < à 50%) des cellules NK CD56+ ont exprimé le CD107 après les 4 h d'incubation. De plus, à l'inverse des pourcentages de lyse présentés dans la figure 1A, les pourcentages de cellules CD56+CD107+ ont diminué avec l'augmentation du rapport E : C. Cependant, le nombre absolu de cellules NK CD107+ calculé à partir du nombre de cellules NK initialement ajouté pour chaque rapport E : C, est bien corrélé au pourcentage de lyse des cellules Daudi pour une préparation de cellules NK provenant d'un donneur donné.

**Exempte 2 : Mesure de l'ADCP exercée par les cellules NK vis-à-vis des cellules Daudi en présence de rituximab par le dosage de l'IFN$\gamma$ sécrété et par la détection des cellules productrices.**

Afin d'étudier si l'ADCP et l'ADCC peuvent être exercées simultanément par les cellules NK, la production d'IFN$\gamma$ par des cellules NK a été évaluée dans les conditions expérimentales précédentes : $10^4$ à $2\times10^5$ cellules NK purifiées d'un même donneur ont été incubées pendant 4h à 37°C en présence de 2 $\mu$g/ml de rituximab avec $2\times10^4$ cellules Daudi non marquées. Le dosage de l'IFN$\gamma$ a été réalisé dans le surnageant par une technique ELISA. Comme le montre la figure 2A et comme cela était le cas pour les pourcentages de lyse, la concentration d'IFN$\gamma$ dans le surnageant a augmenté avec le rapport E : C. Afin de déterminer si la totalité des cellules synthétisent l'IFN$\gamma$, les cellules NK provenant du même donneur ont été incubées dans des conditions expérimentales identiques, mais en présence de brefeldine A qui bloque les mécanismes de transport cellulaire, et donc la sécrétion. Les cellules ont ensuite été marquées avec un AcMo anti-CD56 conjugué avec le PC5, perméabilisées et incubées avec un AcMo anti-IFN$\gamma$ conjugué avec le PE, et le pourcentage de cellules NK exprimant l'IFN$\gamma$ intracellulaire a été évalué par cytométrie en flux d'après les techniques publiées (Mendes et al., 2000; Vitale et al., 2000). En absence de brefeldine A, aucune cellule CD56+IFNγ+ n'a été détectée (résultats non figurés). En revanche, la figure 2B montre qu'une fraction des cellules CD56+ était IFNγ+ après les 4 h d'incubation en présence de brefeldine A. Cependant, cette fraction était relativement faible (dans tous les cas < à 25%). De plus, à l'inverse des concentrations d'IFNγ (figure 2A), les pourcentages de cellules CD56+ IFNγ+ ont diminué quand le rapport E : C a augmenté entre 1,25 : 1 et 10 : 1. Cependant, le nombre absolu de cellules NK IFNγ+ calculé à partir du nombre de cellules NK initialement ajouté pour chaque rapport E : C en présence de brefeldine A, est bien corrélé à la concentration d'IFNγ produite par les cellules NK provenant du même donneur en absence de brefeldine A.

**Exemple 3: Étude simultanée de la dégranulation et de la synthèse d' IFNγ par les cellules NK stimulées par des cellules Daudi en présence de rituximab ou de 3G8**

Les résultats de la figure 1B et 2B montrent que, dans les conditions expérimentales utilisées, ni l'ADCC ni l'ADCP ne sont exercées par la totalité des cellules NK. Afin d'étudier si les 2 réponses sont exercées, au moins en partie par les mêmes cellules effectrices, il était nécessaire d'étudier simultanément l'expression du CD107 et de l'IFNγ intracellulaire, ce qui ne pouvait être réalisé qu'en présence de brefeldine A. Afin d'étudier l'éventuelle influence de la brefeldine sur l'expression du CD107, des cellules NK purifiées ont été incubées dans les conditions de la figure 1A avec des cellules Daudi en présence de 2 μg/ml de rituximab, en présence de l'AcMo anti-CD107 et en présence ou en absence de brefeldine, puis marquées par l'AcMo anti-CD56 et analysées par cytométrie en flux. Comme le montre la figure 3, les pourcentages de cellules CD56+ CD107+ obtenus en absence ou en présence de brefeldine A étaient similaires (histogrammes blancs versus histogrammes avec des points).

Le résultat précédent permettait donc d'étudier simultanément la dégranulation et la synthèse d'IFNγ par les cellules NK. Pour cela, des cellules NK purifiées ont été incubées pendant 4h à 37°C avec des cellules Daudi en présence de brefeldine A, de l'AcMo anti-CD107 et en absence ou en présence de 2 μg/ml de rituximab ou de l'AcMo murin anti-CD16 3G8. Ce dernier est utilisé pour réaliser un test de stimulation redirigée. En effet, les cellules Daudi expriment la molécule CD32/FcγRII qui possède une affinité pour la portion Fc des AcMo murin d'isotype IgG1 comme le 3G8. Dans ces conditions, l'AcMo 3G8 fixé sur la cible peut recruter par son site de reconnaissance les cellules NK qui expriment CD16/FcγRIIIa. Cette fixation de l'AcMo sur le FcγRIIIa/CD16 active la cellule effectrice et induit sa réponse vis-à-vis de la cible. La forte affinité du 3G8 pour le FcγRIIIa/CD16 permet une stimulation optimale (Dall'Ozzo *et al.,* 2004). Les cellules ont ensuite été marquées par l'AcMo anti-CD56, perméabilisées et incubées avec l'AcMo anti-IFNγ puis analysées par cytométrie en flux.

Les cytogrammes de la figure 4 montrent qu'aucune cellule NK CD107+ ou IFNγ+ n'a été détectée en absence de stimulant (cytogramme A). Au contraire, des pourcentages significatifs de cellules NK CD107+ et de cellules NK IFNγ+ sont observés après stimulation en présence de rituximab ou de 3G8 (cytogramme B et C). Si une fraction des cellules NK est à la fois CD107+ et IFNγ+ (quadrant supérieur droit), montrant que l'ADCC et l'ADCP ne sont pas mutuellement exclusives, la majorité des cellules répondeuses est exclusivement CD107+ (quadrant supérieur gauche) ou exclusivement IFNγ+ (quadrant inférieur droit). Les résultats obtenus avec le rituximab et le 3G8 étaient comparables. Il est à noter que dans ces conditions expérimentales, les cellules répondeuses ne représentaient qu'une fraction minoritaire des cellules NK.

**Exemple 4: Étude simultanée de la dégranulation et de la synthèse d' IFNγ par les cellules NK stimulées par le PMA et le CaI**

Les résultats précédents ont montré qu'en réponse à l'engagement du FcγRIIIa par le rituximab ou le 3G8, les cellules NK qui dégranulent et celles qui synthétisent l'IFNγ appartiennent à des populations au moins en partie différentes. Afin d'étudier si cette dichotomie fonctionnelle des cellules NK pouvait être observée dans des conditions de stimulation indépendantes du CD16, des cellules NK purifiées ont été stimulées par différentes concentrations de l'ionophore calcique (CaI) A23187 et de phorbol myristate acetate (PMA) utilisées seules ou en association. En effet, il a été rapporté que la stimulation par cette combinaison permettait d'une part la dégranulation des cellules NK (Atkinson et al., 1990) et d'autre part la synthèse de cytokines (Mendes et al., 2000). L'activation a été réalisée dans une plaque de culture, où les cellules NK ont été incubées pendant 4h à 37°C sous $CO_2$, en présence de l'AcMo anti-CD107, puis les cellules ont été perméabilisées, marquées avec l'AcMo anti-IFNγ et analysées par cytométrie en flux.

Aucune cellule NK IFNγ+ n'a été détecté après stimulation par le CaI en absence de PMA ou par le PMA en absence de CaI (Figure 5). Inversement, la stimulation des cellules par des concentrations de CaI supérieure à 100 ng/ml a induit l'expression du CD107 sur les NK. L'expression était maximale (autour de 15% de cellules CD107+) dès la concentration de 330 ng/ml de CaI. De même, la stimulation par des concentrations de 3,3 à 100 ng/ml de PMA a induit l'expression du CD107 sur environ 10% des cellules NK. Quand les cellules NK ont été stimulées par la combinaison des deux stimulants, le profil d'expression du CD107 comme celui de l'IFNγ a été identique pour toutes les concentrations de PMA utilisées (de 3,3 à 100 ng/ml). Ainsi, l'effet du CaI a été synergique avec celui du PMA pour des concentrations de CaI supérieures à 100 ng/ml tant pour l'expression du CD107 que pour la synthèse d'IFNγ et les deux réponses ont été maximales à la concentration de 330 ng/ml. En revanche, l'expression du CD107 est restée maximale pour des concentrations de 1000 et 3300 ng/ml alors que la synthèse d'IFNγ a diminué sensiblement à ces mêmes concentrations. Enfin, comme précédemment, trois populations de cellules répondeuses ont été observées dans toutes les conditions de stimulation ; des cellules CD107+, des cellules IFNγ+ et des cellules CD107+IFNγ+. Ces dernières n'ont représenté qu'une fraction minoritaire de la totalité des cellule répondeuses, soit de 10 à 20% des cellules CD107+ mais généralement plus de 50% des cellules IFNγ+.

La cinétique d'expression du CD107 et de l'IFNγ intracellulaire a également été étudiée après stimulation des cellules par une combinaison de 330 ng/ml de CaI et 33 ng/ml de PMA. Les cellules NK purifiées ont été stimulées en présence de bréfeldine A et d'AcMo anti-CD107. Après des temps d'incubation compris entre 0 et 4h, les cellules ont été perméabilisées puis marquées avec l'AcMo anti IFNγ et analysées par cytométrie en flux. La figure 6 montre que l'expression du CD107 sur les cellules NK était déjà importante après la première heure de stimulation et que les pourcentages ont ensuite augmenté régulièrement avec le temps. Les cellules NK IFNγ+ ont été détectées après 2h de stimulation et les pourcentages obtenus n'ont pas augmenté après des temps de stimulation supérieurs.

**Exemple 5: Étude simultanée de la perte du CD16, de la dégranulation et de la synthèse d'IFNγ par les cellules NK stimulées par un anticorps anti-CD16 immobilisé sur un support plastique**

Pour étudier l'effet de l'engagement du seul FcγRIIIa sur les réponses des cellules NK, des cellules NK purifiées ont été incubées pendant 4h à 37°C en présence de bréfeldine A et de l'AeMo anti-CD107 dans des puits d'une plaque de culture sur lesquelles avaient été préalablement adsorbées des quantités variables de l'AcMo anti-CD16 3G8. Les quantités de 3G8 effectivement immobilisées au fond des puits de la plaque de culture ont été évaluées par une technique ELISA en utilisant un anticorps anti-IgG de souris marqué par une enzyme. Les résultats sont présentés en valeurs d'absorbance (Figure 7 : ordonnée gauche) par rapport à la concentration de 3G8 utilisée lors de l'adsorption (Figure 7 : abscisse). Les cellules ont ensuite été marquées avec un AcMo anti-CD16 conjugué au FITC. En effet, il a été rapporté que le pontage du CD16 par un AcMo anti-CD16 se traduisait par une perte d'expression membranaire de la molécule sur les cellules NK (Borrego et al., 1994). Les cellules ont finalement été perméabilisées, incubées avec l'AcMo anti

IFNγ et analysées par cytométrie en flux. Les résultats sont exprimés en pourcentage de cellules positives (Figure 7 : ordonnée droite).

La figure 7 montre que la saturation des puits de la plaque par l'AcMo 3G8 a été obtenue pour une concentration de l'ordre de 1 $\mu$g/ml. Après incubation des cellules NK dans les puits sensibilisés par les différentes concentrations de l'AcMo 3G8, une diminution de l'expression membranaire du CD16 a été observée à partir de 0,1 $\mu$g/ml de 3G8 qui représente la concentration entraînait environ 50% de la saturation. L'expression membranaire du CD16 a ensuite diminué très fortement avec l'augmentation de la concentration (environ 15% de cellules positives à 0,3 $\mu$g/ml correspondant à environ 85% de la saturation) pour devenir indétectable aux concentrations saturantes. Ainsi, près de 100% des cellules NK ont perdu l'expression du CD16 et ont donc été activées, après incubation dans les puits sensibilisés par une concentration saturante de 3G8. Des cellules NK CD107+ et/ou IFNγ+ ont été détectées après incubation dans des puits sensibilisés par une concentration de 0,1 $\mu$g/mL. Les pourcentages de cellules NK CD107+ et/ou IFNγ+ ont ensuite augmenté avec l'augmentation de la concentration de 3G8 pour atteindre un plateau pour une concentration de 0,3 $\mu$g/ml. Les trois populations de cellules répondeuses ont à nouveau été observées dans ces conditions de stimulation.

**Exemple 6 : Etude comparative de la sensibilisation des plaques par plusieurs AcMor thérapeutiques et par des IgG humaines**

Pour pouvoir comparer les réponses fonctionnelles induites par les différents AcMor adsorbés sur un support plastique, il était nécessaire de comparer au préalable leur adsorption sur celui-ci. Les puits des microplaques ont donc été sensibilisées pendant une nuit 4°C avec des concentrations comprises entre 0.01 et 10 $\mu$g/mL d'AcMor ou d'IgG humaine de chacune des 4 sous-classes ou d'IgG humaine polyvalente ou d'etanercept (protéine de fusion comprenant une portion Fc). La fixation a été révélée par ELISA à l'aide d'un Ac de chèvre anti-Fc humain couplé à la peroxydase. La figure 8 montre, pour chacun des produits testés, le profil de l'absorbance et donc de la fixation en fonction de la concentration sensibilisante. Comme attendu, la fixation de l'abciximab, qui ne possède pas de portion Fc, n'a pas été détectée dans ce système expérimental. En revanche, pour tous les AcMor ou IgG testés, la fixation a augmenté avec la concentration sensibilisante jusqu'à un plateau selon un profil sigmoïde. Les courbes obtenues avec l'IgG3, l'IgG4 et à un moindre degré avec l'IgGI étaient décalées vers la droite, indiquant une sensibilisation un peu moins importante avec ces protéines myélomateuses qu'avec les AcMor. Inversement, la courbe obtenue avec le palivizumab était légèrement décalée vers la gauche, montrant une meilleure sensibilisation de la plaque avec cet AcMor. Les résultats obtenus avec les autres produits étaient similaires. Pour tous les produits testés à l'exception du palivizumab, la saturation de la plaque a été obtenue avec des concentrations sensibilisantes comprises entre 1 et 3 $\mu$g/mL.

**Exemple 7: Etude comparative de la fixation du C1q par plusieurs AcMor thérapeutiques et par des IgG humaines.**

Afin de confirmer que la fixation des différents AcMor ou IgG humaines était équivalente aux concentrations sensibilisantes saturantes, les plaques ont été sensibilisées avec 5 $\mu$g/mL de chacun des AcMor ou d'IgG humaine puis incubées avec des concentrations comprises entre 0.01 et 10 $\mu$g/mL de C1q humain qui se fixe sur le domaine CH2 de la portion Fc. La fixation du C1q a été révélée par ELISA à l'aide d'un Ac de mouton anti-C1q humain couplé à la peroxydase. La figure 9 montre pour chacun des produits testés le profil de l'absorbance et donc de la fixation du C1q en fonction de la concentration de celui-ci. Comme attendu, le C1q ne s'est pas fixé sur l'abciximab qui ne possède pas de portion Fc, ni sur le 3G8 qui est un AcMo murin non reconnu par le C1q humain. En revanche, une importante fixation a été observée avec tous les autres AcMor ou IgG testés. Celle-ci a augmenté avec la concentration jusqu'à un plateau selon un profil sigmoïde. Tous les profils obtenus étaient identiques, démontrant que quelle que soit l'AcMor ou l'IgG humaine utilisé, la concentration sensibilisante de 5 $\mu$g/mL permet une saturation effective de la plaque. Ces résultats montrent également qu'il n'existe pas de variabilité entre les AcMor testés pour la fixation du C1q et que celle-ci ne nécessite pas que l'Ac soit fixé sur l'Ag dont il est spécifique.

**Exemple 8 : Etude comparative de l'affinité de plusieurs AcMor thérapeutiques et des IgG humaines pour le Fc$\gamma$RIIIa des cellules NK.**

La réponse fonctionnelle des cellules exprimant le Fc$\gamma$RIIIa vis-à-vis d'une cible sensibilisée par un AcMor dépend de l'affinité du récepteur pour l'AcMor (Dall'Ozzo et al., 2004). Afin de comparer la réponse fonctionnelle induite par différents AcMor adsorbés sur la plaque il était nécessaire de comparer leur affinité pour le Fc$\gamma$RIIIa exprimé sur les cellules répondeuses. Pour cela, la capacité de concentrations comprises entre 0.001 et 10 mg/mL de chacun des AcMor ou d'IgG polyvalente, d'inhiber la fixation de l'AcMo anti-CD16 3G8 conjugué au FITC sur les cellules NK purifiées d'un donneur a été étudiée par cytométrie en flux comme décrit précédemment (Dall'Ozzo et al., 2004). La figure 10 montre, pour chacun des produits testés, l'inhibition de la fixation du 3G8 en fonction de la concentration du produit testé. Pour tous les AcMor testés et pour l'IgG polyvalente, l'inhibition de la fixation du 3G8 a augmenté avec la concentration jusqu'à un plateau selon un profil sigmoïde. Cependant, les pourcentages d'inhibition observés dépendaient fortement du produit testé. Ainsi, la concentration C1 d'infliximab permettant d'inhiber 50% de la fixation du 3G8 est environ 20 fois inférieure à la concentration C2 de basiliximab produisant le même effet, traduisant la meilleure affinité du premier pour le Fc$\gamma$RIIIa exprimé sur les cellules NK. Au total, bien que les AcMor thérapeutiques commercialisés soient tous des IgGl et qu'ils possèdent donc une portion Fc de séquence conservée, ils présentent des affinités variables pour le Fc$\gamma$RIIIa.

**Exemple 9 : Etude comparative de la perte du CD16, de la dégranulation et de la synthèse d'IFN$\gamma$ par les cellules NK incubées dans des plaques sensibilisées en fonction des AcMor ou des IgG sensibilisant le support plastique.**

Pour comparer les réponses des cellules NK aux différents AcMor, aux IgG humaines, au sérum anti-lymphocytaire et à l'AcMo anti-CD16 3G8, les cellules NK purifiées ont été incubées pendant 4h à 37°C en présence de brefeldine A et de l'AcMo anti-CD107 dans des puits d'une plaque de culture préalablement sensibilisées ou non par une concentration saturante ($\geq$ 3$\mu$g/mL) de chacun des AcMor ou d'IgG polyvalente ou d'IgGl ou d'IgG3 ou de sérum anti-lymphocytaire ou de l'ACMo anti-CD16 3G8. Les cellules ont ensuite été marquées avec un AcMo anti-CD16. Les cellules ont finalement été perméabilisées, incubées avec l'AcMo anti IFN$\gamma$ et analysées par cytométrie en flux. La figure 11 montre pour chacun des produits testés le pourcentage moyen des cellules NK ayant eu une perte d'expression membranaire du CD16 d'une part et les pourcentages moyens de cellules CD107+ et/ou IFN$\gamma$+ d'autre part (résultats classés par ordre croissant de perte du CD16 et obtenus au cours de 8 expériences différentes). Comme cela a été montré précédemment dans la figure 7, les cellules NK incubées dans des puits non sensibilisés n'ont pas perdu de manière détectable le CD16 et n'ont pas exprimé le CD107 ou l'IFN$\gamma$ intracellulaire. Il en a été de même pour les cellules NK incubées dans des puits sensibilisés par l'abciximab. Pour tous les autres produits les deux réponses ont été détectées. Pour chaque produit testé, le pourcentage des cellules ayant eu une la réponse fonctionnelle (somme des pourcentages des cellules CD107+, CD107+IFN$\gamma$+ et IFN$\gamma$+) a toujours été inférieur au pourcentage des cellules activées (pourcentage des cellules ayant perdu le CD16). Cependant pour l'ensemble des produits testés, les pourcentages des cellules ayant eu une réponse fonctionnelle étaient très bien corrélés aux pourcentages des cellules activées. Comme cela a été montré précédemment dans la figure 7, près de 100% des cellules NK incubées dans des puits sensibilisés par l'AcMo anti-CD16 3G8 ont perdu l'expression du CD16 et ont donc été activées. Environ 1/3 des cellules NK ont également eu une réponse fonctionnelle, parmi lesquelles une majorité (près de 85%) exprimait le CD107 seul ou associé à l'IFNγ intracellulaire. Des résultats similaires ont été observés avec les cellules NK incubées dans des puits sensibilisés par le sérum anti-lymphocytaire. Les cellules NK incubées dans des puits sensibilisés par l'IgG3 humaine ont eu une perte de CD16 et une réponse fonctionnelle très faibles. Les réponses (perte du CD16 et réponses fonctionnelles déterminées par le CD107 et l'IFNγ intracellulaire) des cellules NK incubées dans des puits sensibilisés par des AcMor ont varié sensiblement selon L'AcMo utilisé. Le basiliximab a induit les réponses les plus faibles (en moyenne un peu plus de 20% de cellules ayant perdu le CD16), puis le cetuximab et le rituximab (en moyenne un peu moins de 30% de cellules ayant perdu le CD16), puis le trastuzumab, le palivizumab, l'adalimumab, l'infliximab et le bevacizumab ont entraîné des réponses plus fortes proches de celles obtenus avec l'IgG polyvalente et l'IgG1 humaine (en moyenne entre 36.5% et 43.4% de cellules ayant perdu le CD16). Enfin, l'alemtuzumab a induit une réponse massive proche de celle observée avec le 3G8 (en moyenne 78.5% de cellules ayant perdu le CD16). Parmi les cellules NK ayant eu une réponse fonctionnelle, une majorité exprimait le CD107 seul ou associé à l'IFNγ. Cependant la proportion de cellules CD107+ était inférieure (comprise entre 58% et 72%) à celle observée après incubation des cellules dans des puits sensibilisés par l'Anti-CD16 3G8 ou le sérum anti-lymphocytaire. Au total, la sensibilisation des plaques par une concentration saturante de différents AcMor a entraîné une activation et des réponses fonctionnelles variables de la part des cellules NK en fonction de l'AcMo utilisé. Ces résultats montrent donc qu'indépendamment des antigènes qu'ils reconnaissent, les différents AcMor activent différemment, par leur portion Fc, les cellules NK et que la méthode utilisée permet de mesurer et de comparer les réponses produites par ces différents AcMor.

**Exemple 10 : Etude comparative de la perte du CD16, de la dégranulation et de la synthèse d'IFNγ par les cellules NK incubées dans des plaques sensibilisées en fonction du donneur de cellules.**

Les résultats individuels de l'activation et de la réponse fonctionnelle de 3 parmi les 8 expériences de la figure 11 sont présentés dans la figure 12. Conformément aux résultats montré dans la figure 11, les cellules NK des 3 donneurs (figures 12A, 12B et 12C) ont eu des réponses nulles ou très faibles après incubation dans des puits non sensibilisées ou sensibilisés avec l'abciximab et ont présentés des réponses très importantes après incubation dans les puits sensibilisés avec l'AcMo anti-CD16 3.G8, le sérum anti-lymphocytaire et l'alemtuzumab. Les réponses aux autres produits testés ont fortement varié avec le donneur. Ainsi, les cellules NK du premier donneur (Figure 12A) ont eu une perte du CD16 très inférieure à la moyenne (de 1% pour l'IgG3 à 27% pour l'IgGl) et des réponses fonctionnelles également très faibles. Les cellules NK du deuxième donneur ont eu une perte du C16 très supérieure à la moyenne (de 17% pour l'IgG3 à 82% pour l'IgGl) et des réponses fonctionnelles intermédiaires. De plus, parmi les cellules NK de ce donneur ayant eu une réponse fonctionnelle, une minorité exprimait le CD107 seul ou associé à l'IFNγ, y compris après stimulation par l'alemtuzumab. Enfin, les cellules NK du troisième donneur ont eu une perte du CD16 (de 22% pour l'IgG3 à 57% pour l'IgGl) et des réponses fonctionnelles légèrement supérieures à la moyenne. Parmi les cellules NK de ce donneur ayant eu une réponse fonctionnelle, une large majorité exprimait le CD107 seul ou associé à l'IFNγ quel que soit le produit testé. Au total, l'activation et les réponses fonctionnelles des cellules NK, après incubation dans des plaques sensibilisées par une concentration saturante de certains AcMor ou d'IgG humaine, varient qualitativement et quantitativement en fonction du donneur des cellules. Ces résultats montrent donc que les réponses à la portion Fc de certains AcMor ou IgG humaines dépendent de l'origine des cellules NK, et que la méthode utilisée permet de mesurer et de comparer les réponses produites par les cellules NK de différents donneurs.

**Exemple 11 : Etude comparative de la dégranulation et de la synthèse d'IFNγ par les cellules NK et par les PBMC d'un même donneur incubés dans des plaques sensibilisées.**

Afin de vérifier que la méthode est utilisable sans étape de purification des cellules NK, les PBMC et les cellules NK d'un même donneur ont été préparées et incubées pendant 4h à 37°C en présence de brefeldine A et de l'AcMo anti-CD107 dans des puits d'une plaque de culture préalablement sensibilisée ou non par une concentration saturante de rituximab, d'infliximab, d'alemtuzumab ou de l'AcMo anti-CD16 3G8. Les cellules ont ensuite été marquées avec un AcMo anti-CD56. Les cellules ont finalement été perméabilisées, incubées avec l'AcMo anti IFNγ et analysées par cytométrie en flux. La figure 13 montre pour chacun des produits testés, les pourcentages de cellules CD107+ et/ou IFNγ+ parmi les cellules NK CD56+ et parmi les PBMC CD56+. Les pourcentages de cellules répondeuses obtenus avec les cellules NK purifiées et avec les PBMC CD56+ ont été comparables.

**Exemple 12 : Etude de la perte du CD16, de la dégranulation et de la synthèse d'IFNγ par les cellules NK incubées dans des plaques co-sensibilisées par le 3G8 ou l'infliximab et par des AcMo dirigés contre des molécules d'activation ou de co-activation des cellules NK.**

Il a été montré que les récepteurs NKp30, NKp46, NKG2D étaient impliqués dans la cytotoxicité naturelle (Pende et al., 2001), et que des récepteurs comme CD2, 2B4 (CD244) ou LFA-1 (CD11a/CD18) intervenaient dans la co-activation de la cellule NK (Bryceson et al., 2006; Natarajan et al., 2002). De plus, NKG2D semble avoir un rôle dans la synthèse naturelle de cytokine par les cellules NK (Andre et al., 2004). De même, la molécule membranaire CD45 semble être indispensable à la production de cytokines par les cellules NK de souris mais pas à la cytotoxicité exercée par ces mêmes cellules (Hesslein et al., 2006; Huntington et al., 2005). Afin d'étudier l'effet de la stimulation de ces molécules simultanément à l'engagement du FcγRIIIa sur l'orientation fonctionnelle des cellules NK, des plaques sensibilisées par des anticorps dirigés contre ces molécules ou contre la molécule d'adhésion C56 (qui ne possède pas d'activité co-activatrice) ou contre une IgGl murine et co-sensibilisées ou non par l'anticorps anti-CD16 3G8 ou par l'infliximab (1,5 μg/mL) ont été utilisées. La stimulation et les marquages ont été réalisés comme cela a été décrit dans l'exemple 9. La figure 14A montre les pourcentages de cellules NK CD16+, CD107+ et IFNγ+ d'un donneur après incubation dans des plaques non sensibilisées ou uniquement sensibilisées par l'IgGl contrôle ou par les AcMo dirigés contre les molécules CD56, NKG2D, NKP30, CD11a CD244, CD45 ou CD2. Les cellules NK incubées dans ces conditions n'ont pas perdu de manière détectable le CD16 et n'ont exprimé ni le CD107 (à l'exception d'une très faible expression après incubation en présence de l'AcMo anti-NKP30) ni l'IFNγ intracellulaire. Au contraire la quasi-totalité des cellules NK incubées dans des plaques sensibilisées par l'AcMo 3G8 (Figure 14B) ont eu une perte d'expression du CD16, que les plaques aient été co-sensibilisées ou non par les différents AcMo dirigés contre les molécules d'activation testées. En accord avec ce résultat et avec ceux présentés dans les figures 11 et 12, une fraction significative de ces cellules était CD107+ (de 25 à 33%) ou IFNγ+ (de 9 à 13%). Cependant, les réponses n'ont pas été sensiblement modifiées par le co-engagement des molécules d'activation ou de co-activation testées. Enfin, comme le montre la figure 14C, une fraction significative des cellules NK de ce donneur incubées dans des plaques sensibilisées par l'infliximab a eu une perte d'expression du CD16 (de 47% des cellules incubées dans les puits sensibilisés par l'infliximab et l'IgGl murine contrôle à 68% des cellules incubées dans des puits co-sensibilisés par l'infliximab et l'AcMo anti-CD45). En accord avec ce résultat et avec ceux présentés dans les figures 11 et 12, une fraction significative de ces cellules était CD107+ ou IFN-γ+, mais les pourcentages ont varié sensiblement en fonction des molécules d'activation ou de co-activation testées. Ainsi, 13,6% des cellules NK étaient CD107+ après incubation dans des puits co-sensibilisés par l'IgGl murine contre environ 20% des cellules incubées dans des puits co-sensibilisés par les AcMo dirigés contre CD56, CU11a, CD244, CD45 et CD2 et environ 30% des cellules incubées dans des puits co-sensibilisés par les AcMo dirigés contre NKG2D et NKP30. De même, de 6,1 à 9% des cellules NK étaient IFN-γ+ après incubation dans des puits co-sensibilisés par l'IgGl murine, ou par les AcMo dirigés contre CD56, NKG2D, CD45 et CD2 contre seulement 2% des cellules incubées dans des puits co-sensibilisés par l'AcMo anti-CD11a et contre 12 à 13% des cellules incubées dans des puits co-sensibilisés par les AcMo anti-NKP30 et anti-CD244. Ainsi, le co-engagement de la molécule NKP30 a augmenté les deux réponses fonctionnelles induites par l'infliximab, celui de NKG2D a uniquement augmenté la dégranulation, celui de CD244 a uniquement augmenté la synthèse d'IFN-γ, alors que celui de CD11a a diminué cette dernière. Au total, ces résultats montrent donc que, dans nos conditions expérimentales, l'engagement des seules molécules d'activation ou de co-activation des cellules NK n'a pas induit de réponse détectable. En revanche, le co-engagemént des molécules d'activation ou de co-activation des cellules NK simultanément à celui du FcγRIIIa par l'infliximab (mais pas par le 3G8) a entraîné des variations quantitatives et qualitatives des réponses fonctionnelles de ces cellules.

**REFERENCES**

Alter, G., Malenfant, J. M., and Altfeld, M. (2004). CD107a as a functional marker for the identification of natural killer cell activity. J Immunol Methods 294, 15-22.

Andre, P., Castriconi, R., Espeli, M., Anfossi, N., Juarez, T., Hue, S., Conway, H., Romagne, F., Dondero, A., Nanni, M., et al. (2004). Comparative analysis of human NK cell activation induced by NKG2D and natural cytotoxicity receptors. Eur J Immunol 34, 961-971.

Anegon, I., Cuturi, M. C., Trinchieri, G., and Perussia, B. (1988). Interaction of Fc receptor (CD 16) ligands induces transcription of interleukin 2 receptor (CD25) and lymphokine genes and expression of their products in human natural killer cells. J Exp Med 167, 452-472.

Atkinson, E. A., Gerrard, J. M., Hildes, G. E., and Greenberg, A. H. (1990). Studies of the mechanism of natural tiller (NK) degranulation and cytotoxicity. J Leukoc Biol 47, 39-48.

Betts, M. R., Brenchley, J. M., Price, D. A., De Rosa, S. C., Douek, D. C., Roederer, M., and Koup, R. A. (2003). Sensitive and viable identification of antigen-specific CD8+ T cells by a flow cytometric assay for degranulation. J Immunol Methods 281, 65-78.

Betts, M. R., Price, D. A., Brenchley, J. M., Lore, K., Guenaga, F. J., Smed-Sorensen, A., Ambrozak, D. R., Migueles, S. A., Connors, M., Roederer, M., et al. (2004). The functional profile of primary human antiviral CD8+ T cell effector activity is dictated by cognate peptide concentration. J Immunol 172, 6407-6417.

Boehm, U., Klamp, T., Groot, M., and Howard, J. C. (1997). Cellular responses to interferon-gamma. Annu Rev Immunol 15, 749-795.

Borrego, F., Lopez-Beltran, A., Pena, J., and Solana, R. (1994). Downregulation of Fc gamma receptor IIIA alpha (CD16-II) on natural killer cells induced by anti-CD16 mAb is independent of protein tyrosine kinases and protein kinase C. Cell Immunol 158, 208-217.

Brunner, K. T., Mauel, J., Cerottini, J. C., and Chapuis, B. (1968). Quantitative assay of the lytic action of immune lymphoid cells on 51-Cr-labelled allogeneic target cells in vitro; inhibition by isoantibody and by drugs. Immunology 14, 181-196.

Bryceson, Y. T.; March, M. E., Ljunggren, H. G., and Long, E. O. (2006). Synergy among receptors on resting NK cells for the activation of natural cytotoxicity and cytokine secretion. Blood 107, 159-166.

Cartron, G., Dacheux, L., Salles, G., Solal-Celigny, P., Bardos, P., Colombat, P., and Watier, H. (2002). Therapeutic activity of humanized anti-CD20 monoclonal antibody and polymorphism in IgG Fc receptor FcgammaRIIIa gene. Blood 99, 754-758.

Colucci, F., Rosmaraki, E., Bregenholt, S., Samson, S. I., Di Bartolo, V., Turner, M., Vanes, L., Tybulewicz, V., and Di Santo, J. P. (2001). Functional dichotomy in natural killer cell signaling: Vavl-dependent and -independent mechanisms. J Exp Med 193, 1413-1424.

Cooley, S., Burns, L. J., Repka, T., and Miller, J. S. (1999). Natural killer cell cytotoxicity of breast cancer targets is enhanced by two distinct mechanisms of antibody-dependent cellular cytotoxicity against LFA-3 and HER2/neu. Exp Hematol 27, 1533-1541.

Cooper, M. A., Fehniger, T. A., Turner, S. C., Chen, K. S., Ghaheri, B. A., Ghayur, T., Carson, W. E., and Caligiuri, M. A. (2001). Human natural killer cells: a unique innate immunoregulatory role for the CD56(bright) subset. Blood 97, 3146-3151.

Dall'Ozzo, S., Tartas, S., Paintaud, G., Cartron, G., Colombat; P., Bardos, P., Watier, H., and Thibault, G. (2004). Rituximab-dependent cytotoxicity by natural killer cells: influence of FCGR3A polymorphism on the concentration-effect relationship. Cancer Res 64, 4664-4669.

Faroudi, M., Utzny, C., Salio, M., Cerundolo, V., Guiraud, M., Muller, S., and Valitutti, S. (2003). Lytic versus Stimulatory synapse in cytotoxic T lymphocyte/target cell interaction: manifestation of a dual activation threshold. Proc Natl Acad Sci U S A 100, 14145-14150.

Fukuda, M. (1991). Lysosomal membrane glycoproteins, Structure, biosynthesis, and intracellular trafficking. J Biol Chem 266, 21327-21330.

Gajewski, T. F., Goldwasser, E., and Fitch, F. W. (1988). Anti-proliferative effect of IFN-gamma in immune regulation. II. IFN-gamma inhibits the proliferation of murine bone marrow cells stimulated with IL-3, IL-4, or granulocyte-macrophage colony-stimulating factor. J Immunol 141, 2635-2642.

Greenwood, J., Clark, M., and Waldmann, H. (1993). Structural motifs involved in human IgG antibody effector functions. Eur J Immunol 23, 1098-1104.

Hesslein, D. G., Takaki, R., Hermiston, M. L., Weiss, A., and Lanier, L. L. (2006). Dysregulation of signaling pathways in CD45-deficient NK cells leads to differentially regulated cytotoxicity and cytokine production. Proc Natl Acad Sci U S A 103, 7012-7017.

Huntington, N. D., Xu, Y., Nutt, S. L., and Tarlinton, D. M. (2005). A requirement for CD45 distinguishes Ly49D-mediated cytokine and chemokine production from killing in primary natural killer cells. J Exp Med 201, 1421-1433.

Kurago, Z. B., Lutz, C. T., Smith, K. D., and Colonna, M. (1998). NK cell natural cytotoxicity and IFN-gamma production are not always coordinately regulated: engagement of DX9 KIR+ NK cells by HLA-B7 variants and target cells. J Immunol 160, 1573-1580.

Mendes, R., Bromelow, K. V., Westby, M., Galea-Lauri, J., Smith, I. E., O'Brien, M. E., and Souberbielle, B. E. (2000). Flow cytometric visualisation of cytokine production by CD3-CD56+ NK cells and CD3+CD56+ NK-T cells in whole blood. Cytometry 39, 72-78.

Natarajan, K., Dimasi, N., Wang, J., Mariuzza, R. A., and Margulies, D. H. (2002). Structure and function of natural killer cell receptors: multiple molecular solutions to self, nonself discrimination. Annu Rev Immunol 20, 853-885.

Nathan, C. F., Prendergast, T. J., Wiebe, M. E., Stanley, E. R., Platzer, E., Remold, H. G., Welte, K., Rubin, B. Y., and Murray, H. W. (1984). Activation of human macrophages. Comparison of other cytokines with interferon-gamma. J Exp Med 160, 600-605.

Parihar, R., Dierksheide, J., Hu, Y., and Carson, W. E. (2002): IL-12 enhances the natural killer cell cytokine response to Ab-coated tumor cells. J Clin Invest 110, 983-992.

Pende, D., Cantoni, C., Rivera, P., Vitale, M., Castriconi, R., Marcenaro, S., Nanni, M., Biassoni, R, Bottino, C., Moretta, A., and Moretta, L. (2001). Role of NKG2D in tumor cell lysis mediated by human NK cells: cooperation with natural cytotoxicity receptors and capability of recognizing tumors of nonepithelial origin. Eur J Immunol 31, 1076-1086.

Rajagopalan, S., Fu, J., and Long, E. O. (2001). Cutting edge: induction of IFN-gamma production but not cytotoxicity by the killer cell Ig-like receptor KIR2DL4 (CD158d) in resting NK cells. J Immunol 167, 1877-1881.

Reff, M. E., Camer, K., Chambers, K. S., Chinn, P. C., Leonard, J. E., Raab, R., Newman, R. A., Hanna, N., and Anderson, D. R. (1994). Depletion of B cells in vivo by a chimeric mouse human monoclonal antibody to CD20. Blood 83, 435-445.

Shields, R. L., Namenuk, A. K., Hong, K., Meng, Y. G., Rae, J., Briggs, J., Xie, D., Lai, J., Stadlen, A., Li, B., et al. (2001). High resolution mapping of the binding site on human IgGl for Fc gamma RI, Fc gamma RII, Fc gamma RIII, and FcRn and design of IgG1 variants with improved binding to the Fc gamma R J Biol Chem 276, 6591-6604.

Shinkawa, T., Nakamura, K., Yamane, N., Shoji-Hosaka, E., Kanda, Y., Sakurada, M., Uchida, K., Anazawa, H., Satoh, M., Yamasaki, M., et al. (2003). The absence of fucose but not the presence of galactose or bisecting N-acetylglucosamine of human IgGl complex-type oligosaccharides shows the critical role of enhancing antibody-dependent cellular cytotoxicity. J Biol Chem 278, 3466-3473.

Smyth, M. J., Cretney, E., Kelly, J. M., Westwood, J. A., Street, S. E., Yagita, H., Takeda, K., van Dommelen, S. L., Degli-Esposti, M. A., and Hayakawa, Y. (2005). Activation of NK cell cytotoxicity. Mol Immunol 42, 501-510.

Trinchieri, G. (1989). Biology of natural killer cells. Adv Immunol 47, 187-376.

Umana, P., Jean-Mairet, J., Moudry, R., Amstutz, H., and Bailey, J. E. (1999). Engineered glycoforms of an antineuroblastoma IgG1 with optimized antibody-dependent cellular cytotoxic activity. Nat Biotechnol 17, 176-180.

Vitale, M., Caruso, A., Licenziati, S., Rodella, L., Fiorentini, S., Zauli, G., Castelli, F., Manzoli, F. A., and Turano, A. (2000). Differential production of IFN-gamma, analyzed at the single-cell level, by specific subsets of human NK and T cells from healthy and HIV(+) subjects. Cytometry 39, 189-194.

Wing, M. G., Moreau, T., Greenwood, J., Smith, R. M., Hale, G., Isaacs, J., Waldmann, H., Lachmann, P. J., and Compston, A. (1996). Mechanism of first-dose cytokine-release syndrome by CAMPATH 1-H: involvement of CD16 (FcgammaRIII) and CD11a/CD18 (LFA-1) on NK cells. J Clin Invest 98, 2819-2826.

**Revendications**

1. Méthode pour évaluer *in vitro* les fonctions effectrices de cellules exprimant le FcγRIIIa en réponse à un anticorps monoclonal à tester, comportant au moins les étapes suivantes :

   (i) les cellules exprimant le FcγRIIIa sont mises en contact avec ledit anticorps monoclonal, immobilisé sur un support, en présence d'un agent inhibant la sécrétion des cytokines par lesdites cellules ;
   (ii) à titre de contrôle positif de l'activation des cellules exprimant le FcγRIIIa, la même expérience est réalisée en utilisant, à la place de l'anticorps monoclonal à tester, un anticorps monoclonal dirigé contre le récepteur FcγRIIIa ;
   (iii) après un temps d'incubation d'au moins 1 heure, la réponse des cellules exprimant le FcγRIIIa est observée en mesurant la présence du marqueur CD107 à la surface cellulaire, ainsi que la présence d'interféron gamma (IFNγ) intracellulaire et/ou de facteur de nécrose tumorale alpha (TNFα) intracellulaire.

2. Méthode selon la revendication 1, dans laquelle les cellules exprimant le FcγRIIIa sont des cellules NK ou des lymphocytes T.

3. Méthode selon l'une quelconque des revendications 1 à 2, dans laquelle l'incubation est effectuée en présence d'un anticorps monoclonal anti-CD107 couplé à un marqueur.

4. Méthode selon l'une quelconque des revendications 1 à 3, dans laquelle les cellules sont perméabilisées à la fin de l'incubation pour mesurer l'interféron gamma et/ou le TNF alpha en utilisant un anticorps monoclonal anti-IFNγ couplé à un marqueur et/ou un anticorps monoclonal anti-TNFα couplé à un marqueur.

5. Méthode selon l'une quelconque des revendications 1 à 4, comportant en outre une étape de mesure de l'expression du FcγRIIIa/CD16a à la surface des cellules, par cytométrie en flux après marquage desdites cellules à la fin de l'incubation, avec un anticorps monoclonal anti-CD16 couplé à un marqueur.

6. Méthode selon l'une quelconque des revendications 1 à 5, dans laquelle les marqueurs couplés aux anticorps monoclonaux anti-CD107, anti-IFNγ, anti-TNFα, et anti-CD16, sont des fluorochromes distincts.

**7.** Méthode selon l'une quelconque des revendications 1 à 6, comportant en outre, à titre de contrôle positif de la capacité de réponse des cellules exprimant le FcγRIIIa, une étape (ii-bis) de stimulation desdites cellules par un mélange d'un ester de phorbol et d'un ionophore calcique.

**8.** Méthode selon l'une quelconque des revendications 1 à 7, dans laquelle, à l'issue de l'incubation, la séquence suivante est effectuée :

(iv) les cellules exprimant le FcγRIIIa sont marquées avec un anticorps anti-CD 16 couplé à un marqueur,
(v) les cellules exprimant le FcγRIIIa sont perméabilisées, puis marquées avec un anticorps monoclonal anti-IFNγ couplé à un marqueur, et/ou avec un anticorps monoclonal anti-TNFa couplé à un marqueur,
(vi) les cellules ainsi marquées sont analysées par cytométrie en flux.

**9.** Méthode selon l'une quelconque des revendications 2 à 8, dans laquelle, aux étapes (i) et (ii), les cellules NK sont co-stimulées avec des anticorps dirigés contre un ou plusieurs des récepteurs choisis parmi NKp30, NKp46, NKG2D, 2B4, CD2, CD45, DNAM et CD11a.

**10.** Utilisation d'une méthode selon l'une quelconque des revendications 1 à 9, pour prédire la réponse d'un patient à un traitement par un anticorps monoclonal donné.

**11.** Utilisation selon la revendication 10, dans laquelle la méthode est mise en oeuvre avec des cellules exprimant le FcγRIIIa provenant du patient.

**12.** Utilisation d'une méthode selon l'une quelconque des revendications 1 à 9, pour comparer la capacité d'anticorps monoclonaux recombinants différents à induire la cytotoxicité dépendante des anticorps (ADCC) et la production de cytokines dépendante des anticorps (ADCP) par des cellules exprimant le FcγRIIIa.

**13.** Utilisation d'une méthode selon l'une quelconque des revendications 1 à 9, comme outil de recherche pour améliorer l'efficacité des anticorps monoclonaux recombinants à visée thérapeutique.

**14.** Utilisation d'une méthode selon l'une quelconque des revendications 1 à 9, pour effectuer un contrôle qualité d'un échantillon d'anticorps monoclonaux recombinants.

**15.** Utilisation selon l'une quelconque des revendications 12 à 14, dans laquelle la méthode est mise en oeuvre avec des cellules exprimant le FcγRIIIa provenant de sujets sains et/ou de malades.

**16.** Utilisation selon l'une quelconque des revendications 12 à 14, dans laquelle la méthode est mise en ouvre avec une ou plusieurs lignées de cellules NK et/ou de lymphocytes T.

**17.** Trousse utilisable pour la mise en oeuvre d'une méthode selon l'une quelconque des revendications 1 à 9, comprenant au moins les éléments suivants :

- un anticorps monoclonal anti-CD16 couplé à un marqueur,
- un anticorps monoclonal anti-IFNγ couplé à un marqueur et/ou un anticorps monoclonal anti-TNFα couplé à un marqueur, et
- une plaque comportant au moins un puits recouvert d'un anticorps monoclonal dirigé contre le récepteur FcγRIIIa.

**Claims**

**1.** Method for evaluating *in vitro* the effector functions of cells expressing FcγRIIIa in response to a monoclonal antibody to be tested, comprising at least the following steps:

(i) the cells expressing FcγRIIIa are brought into contact with said monoclonal antibody, which is immobilised on a support, in the presence of an agent inhibiting the secretion of cytokines by said cells;
(ii) as positive control for the activation of the cells expressing FcγRIIIa, the same experiment is carried out using, instead of the monoclonal antibody to be tested, a monoclonal antibody directed against the FcγRIIIa receptor;

(iii) after an incubation time of at least one hour, the response of the cells expressing FcγRIIIa is observed by measuring the presence of the CD107 marker at the cell surface as well as the presence of intracellular interferon gamma (IFNγ) and/or of intracellular tumour necrosis factor alpha (TNFα).

2. Method according to claim 1, wherein the cells expressing FcγRIIIa are NK cells or T lymphocytes.

3. Method according to either claim 1 or claim 2, wherein the incubation is carried out in the presence of an anti-CD107 monoclonal antibody coupled to a marker.

4. Method according to any one of claims 1 to 3, wherein the cells are permeabilised at the end of the incubation in order to measure interferon gamma and/or TNF alpha using an anti-IFNγ monoclonal antibody coupled to a marker and/or an anti-TNFα monoclonal antibody coupled to a marker.

5. Method according to any one of claims 1 to 4, further comprising a step in which the expression of FcγRIIIa/CD16a at the cell surface is measured by flow cytometry after labelling of said cells at the end of the incubation with an anti-CD16 monoclonal antibody coupled to a marker.

6. Method according to any one of claims 1 to 5, wherein the markers coupled to the anti-CD107, anti-IFNγ, anti-TNFα and anti-CD16 monoclonal antibodies are distinct fluorochromes.

7. Method according to any one of claims 1 to 6, further comprising, as positive control for the response capacity of the cells expressing FcγRIIIa, a step (ii-a) in which said cells are stimulated by a mixture of a phorbol ester and a calcium ionophore.

8. Method according to any one of claims 1 to 7, wherein, at the end of the incubation, the following sequence is carried out:

(iv) the cells expressing FcγRIIIa are labelled with an anti-CD16 antibody coupled to a marker,
(v) the cells expressing FcγRIIIa are permeabilised and then labelled with an anti-IFNγ monoclonal antibody coupled to a marker and/or with an anti-TNFα monoclonal antibody coupled to a marker,
(vi) the cells so labelled are analysed by flow cytometry.

9. Method according to any one of claims 2 to 8, wherein, in steps (i) and (ii), the NK cells are costimulated with antibodies directed against one or more receptors selected from NKp30, NKp46, NKG2D, 2B4, CD2, CD45, DNAM and CD11a.

10. Use of a method according to any one of claims 1 to 9 for predicting the response of a patient to treatment with a given monoclonal antibody.

11. Use according to claim 10, wherein the method is carried out with cells expressing FcγRIIIa that come from the patient.

12. Use of a method according to any one of claims 1 to 9 for comparing the capacity of different recombinant monoclonal antibodies to induce antibody-dependent cellular cytotoxicity (ADCC) and antibody-dependent cytokine production (ADCP) by cells expressing FcγRIIIa.

13. Use of a method according to any one of claims 1 to 9 as a research tool for improving the effectiveness of recombinant monoclonal antibodies for therapeutic purposes.

14. Use of a method according to any one of claims 1 to 9 for carrying out a quality control of a sample of recombinant monoclonal antibodies.

15. Use according to any one of claims 12 to 14, wherein the method is carried out with cells expressing FcγRIIIa that come from healthy subjects and/or from sick subjects.

16. Use according to any one of claims 12 to 14, wherein the method is carried out with one ore more NK cell and/or T lymphocyte lines.

17. Kit for use in carrying out a method according to any one of claims 1 to 9, comprising at least the following elements:

- an anti-CD16 monoclonal antibody coupled to a marker,
- an anti-IFNγ monoclonal antibody coupled to a marker and/or an anti-TNFα monoclonal antibody coupled to a marker, and
- a plate having at least one well coated with a monoclonal antibody directed against the FcγRIIIa receptor.

**Patentansprüche**

1. Verfahren zur in vitro-Bewertung der Effektorfunktionen von FcγRIIIa exprimierenden Zellen im Ansprechen auf einen zu testenden monoklonalen Antikörper, wobei das Verfahren zumindest die folgenden Schritte umfasst:

   (i) die FcγRIIIa exprimierenden Zellen werden in Gegenwart eines die Sekretion von Zytokinen durch diese Zellen hemmenden Agens mit dem monoklonalen Antikörper in Berührung gebracht, der auf einem Träger immobilisiert ist;
   (ii) als Positivkontrolle für die Aktivierung der FcγRIIIa exprimierenden Zellen wird der gleiche Versuch durchgeführt, indem anstelle des zu untersuchenden monoklonalen Antikörpers ein gegen den FcγRIIIa-Rezeptor gerichteter monoklonaler Antikörper verwendet wird;
   (iii) nach einer Inkubationsdauer von mindestens 1 h wird das Ansprechen der FcγRIIIa exprimierenden Zellen untersucht, indem das Vorhandensein des CD107-Markers an der Zelloberfläche sowie das Vorhandensein von intrazellulärem Gamma-Interferon (IFNγ) und/oder von intrazellulärem Tumornekrosefaktor-Alpha (TNFα) gemessen wird.

2. Verfahren nach Anspruch 1, wobei die FcγRIIIa exprimierenden Zellen NK-Zellen oder T-Lymphozyten sind.

3. Verfahren nach einem der Ansprüche 1 bis 2, wobei die Inkubation in Gegenwart eines an einen Marker gekoppelten monoklonalen anti-CD107 Antikörpers durchgeführt wird.

4. Verfahren nach einem der Ansprüche 1 bis 3, wobei die Zellen am Ende der Inkubation permeabilisiert werden, um Gamma-Interferon und/oder TNFα unter Verwendung eines an einen Marker gekoppelten monoklonalen anti-IFNγ Antikörpers und/oder eines an einen Marker gekoppelten monoklonalen anti-TNFα Antikörpers zu messen.

5. Verfahren nach einem der Ansprüche 1 bis 4, welches ferner einen Schritt des Messens der Expression von FcγRIIIa / CD16a an der Oberfläche der Zellen mittels Durchflusszytometrie umfasst, nachdem die Zellen am Ende der Inkubation mit einem an einen Marker gekoppelten monoklonalen anti-CD16 Antikörper markiert wurden.

6. Verfahren nach einem der Ansprüche 1 bis 5, wobei die an die monoklonalen anti-CD107, anti-IFNγ, anti-TNFα und anti-CD16 Antikörper gekoppelten Marker voneinander verschiedene Fluorochrome sind.

7. Verfahren nach einem der Ansprüche 1 bis 6, welches ferner als Positivkontrolle für die Ansprechfähigkeit der FcγRIIIa exprimierenden Zellen einen Schritt (ii-bis) des Stimulierens dieser Zellen mittels einer Mischung aus einem Phorbolester und einem Calcium-Ionophor umfasst.

8. Verfahren nach einem der Ansprüche 1 bis 7, wobei nach abgeschlossener Inkubation die folgende Sequenz ausgeführt wird:

   (iv) die FcγRIIIa exprimierenden Zellen werden mit einem an einen Marker gekoppelten anti-CD16 Antikörper markiert,
   (v) die FcγRIIIa exprimierenden Zellen werden permeabilisiert und daraufhin mit einem an einen Marker gekoppelten monoklonalen anti-IFNγ Antikörper und/oder mit einem an einen Marker gekoppelten monoklonalen anti-TNFα Antikörper markiert,
   (vi) die solcherart markierten Zellen werden mittels Durchflusszytometrie analysiert.

9. Verfahren nach einem der Ansprüche 2 bis 8, wobei in den Schritten (i) und (ii) die NK-Zellen mit Antikörpern co-stimuliert werden, die gegen einen oder mehrere der Rezeptoren gerichtet sind, welche unter NKp30, NKp46, NKG2D, 2B4, CD2, CD45, DNAM und CD11a ausgewählt sind.

10. Verwendung eines Verfahrens nach einem der Ansprüche 1 bis 9 zum Vorhersagen des Ansprechens eines Patienten auf eine Behandlung mit einem gegebenen monoklonalen Antikörper.

**11.** Verwendung nach Anspruch 10, wobei das Verfahren mit FcγRIIIa exprimierenden Zellen durchgeführt wird, die vom Patienten stammen.

**12.** Verwendung eines Verfahrens nach einem der Ansprüche 1 bis 9, zum Vergleichen der Fähigkeit verschiedener rekombinanter monoklonaler Antikörper zum Induzieren der von den Antikörpern abhängigen Zytotoxizität (*Antibody-Dependent Cellular Cytotoxicity;* ADCC) und der von den Antikörpern abhängigen Produktion von Zytokinen (*Antibody-Dependent Cytokine Production;* ADCP) durch FcγRIIIa exprimierende Zellen.

**13.** Verwendung eines Verfahrens nach einem der Ansprüche 1 bis 9 als Forschungshilfsmittel zum Verbessern der Effektivität der rekombinanten monoklonalen Antikörper zu therapeutischen Zwecken.

**14.** Verwendung eines Verfahrens nach einem der Ansprüche 1 bis 9 zum Durchführen einer Qualitätskontrolle an einer Probe von rekombinanten monoklonalen Antikörpern.

**15.** Verwendung nach einem der Ansprüche 12 bis 14, wobei das Verfahren mit FcγRIIIa exprimierenden Zellen durchgeführt wird, die von gesunden Testpersonen und/oder von Kranken stammen.

**16.** Verwendung nach einem der Ansprüche 12 bis 14, wobei das Verfahren mit einer oder mehreren Linien von NK-Zellen und/oder T-Lymphozyten durchgeführt wird.

**17.** Kit, welcher für die Durchführung eines Verfahrens nach einem der Ansprüche 1 bis 9 verwendbar ist, wobei dieser Kit mindestens die folgenden Bestandteile aufweist:

- einen an einen Marker gekoppelten monoklonalen anti-CD16 Antikörper,
- einen an einen Marker gekoppelten monoklonalen anti-IFNγ Antikörper und/oder einen an einen Marker gekoppelten monoklonalen anti-TNFα Antikörper, und
- eine Platte, welche mindestens eine Vertiefung aufweist, die mit einem gegen den FcγRIIIa-Rezeptor gerichteten monoklonalen Antikörper beschichtet ist.

**A**

**B**

**C**

$R^2 = 0{,}9119$

**Figure 1**

**A**

**B**

**C**

**Figure 2**

**Figure 3**

**Figure 4**

**Figure 5**

EP 2 062 047 B1

Figure 6

28

CD16+ CD107+ IFN+CD107+ IFN+ 3G8

**Figure 7**

Figure 8

**Figure 9**

Figure 10

Figure 11

Pourcentage perte CD16  CD107+ total  107 / IFN +  IFN +

**Figure 12A**

Pourcentage perte CD16  CD107+ total  107 / IFN +  IFN +

**Figure 12B**

Figure 12C

Figure 13

Figure 14

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1298219 A

**Littérature non-brevet citée dans la description**

- **Alter, G. ; Malenfant, J. M. ; Altfeld, M.** CD107a as a functional marker for the identification of natural killer cell activity. *J Immunol Methods,* 2004, vol. 294, 15-22
- **Andre, P. ; Castriconi, R. ; Espeli, M. ; Anfossi, N. ; Juarez, T. ; Hue, S. ; Conway, H. ; Romagne, F. ; Dondero, A. ; Nanni, M. et al.** Comparative analysis of human NK cell activation induced by NKG2D and natural cytotoxicity receptors. *Eur J Immunol,* 2004, vol. 34, 961-971
- **Anegon, I. ; Cuturi, M. C. ; Trinchieri, G. ; Perussia, B.** Interaction of Fc receptor (CD 16) ligands induces transcription of interleukin 2 receptor (CD25) and lymphokine genes and expression of their products in human natural killer cells. *J Exp Med,* 1988, vol. 167, 452-472
- **Atkinson, E. A. ; Gerrard, J. M. ; Hildes, G. E. ; Greenberg, A. H.** Studies of the mechanism of natural tiller (NK) degranulation and cytotoxicity. *J Leukoc Biol,* 2000, vol. 47, 39-48
- **Betts, M. R. ; Brenchley, J. M. ; Price, D. A. ; De Rosa, S. C. ; Douek, D. C. ; Roederer, M. ; Koup, R. A.** Sensitive and viable identification of antigen-specific CD8+ T cells by a flow cytometric assay for degranulation. *J Immunol Methods,* 2003, vol. 281, 65-78
- **Betts, M. R. ; Price, D. A. ; Brenchley, J. M. ; Lore, K. ; Guenaga, F. J. ; Smed-Sorensen, A. ; Ambrozak, D. R. ; Migueles, S. A. ; Connors, M. ; Roederer, M. et al.** The functional profile of primary human antiviral CD8+ T cell effector activity is dictated by cognate peptide concentration. *J Immunol,* 2004, vol. 172, 6407-6417
- **Boehm, U. ; Klamp, T. ; Groot, M. ; Howard, J. C.** Cellular responses to interferon-gamma. *Annu Rev Immunol,* 1997, vol. 15, 749-795
- **Borrego, F. ; Lopez-Beltran, A. ; Pena, J. ; Solana, R.** Downregulation of Fc gamma receptor IIIA alpha (CD16-II) on natural killer cells induced by anti-CD16 mAb is independent of protein tyrosine kinases and protein kinase C. *Cell Immunol,* 1994, vol. 158, 208-217

- **Brunner, K. T. ; Mauel, J. ; Cerottini, J. C. ; Chapuis, B.** Quantitative assay of the lytic action of immune lymphoid cells on 51-Cr-labelled allogeneic target cells in vitro; inhibition by isoantibody and by drugs. *Immunology,* 1968, vol. 14, 181-196
- **Bryceson, Y. T. ; March, M. E. ; Ljunggren, H. G. ; Long, E. O.** Synergy among receptors on resting NK cells for the activation of natural cytotoxicity and cytokine secretion. *Blood,* 2006, vol. 107, 159-166
- **Cartron, G. ; Dacheux, L. ; Salles, G. ; Solal-Celigny, P. ; Bardos, P. ; Colombat, P. ; Watier, H.** Therapeutic activity of humanized anti-CD20 monoclonal antibody and polymorphism in IgG Fc receptor FcgammaRIIIa gene. *Blood,* 2002, vol. 99, 754-758
- **Colucci, F. ; Rosmaraki, E. ; Bregenholt, S. ; Samson, S. I. ; Di Bartolo, V. ; Turner, M. ; Vanes, L. ; Tybulewicz, V. ; Di Santo, J. P.** Functional dichotomy in natural killer cell signaling: Vavl-dependent and -independent mechanisms. *J Exp Med,* 2001, vol. 193, 1413-1424
- **Cooley, S. ; Burns, L. J. ; Repka, T. ; Miller, J. S.** Natural killer cell cytotoxicity of breast cancer targets is enhanced by two distinct mechanisms of antibody-dependent cellular cytotoxicity against LFA-3 and HER2/neu. *Exp Hematol,* 1999, vol. 27, 1533-1541
- **Cooper, M. A. ; Fehniger, T. A. ; Turner, S. C. ; Chen, K. S. ; Ghaheri, B. A. ; Ghayur, T. ; Carson, W. E. ; Caligiuri, M. A.** Human natural killer cells: a unique innate immunoregulatory role for the CD56(bright) subset. *Blood,* 2001, vol. 97, 3146-3151
- **Dall'Ozzo, S. ; Tartas, S. ; Paintaud, G. ; Cartron, G. ; Colombat; P. ; Bardos, P. ; Watier, H ; Thibault, G.** Rituximab-dependent cytotoxicity by natural killer cells: influence of FCGR3A polymorphism on the concentration-effect relationship. *Cancer Res,* 2004, vol. 64, 4664-4669

- **Faroudi, M. ; Utzny, C. ; Salio, M. ; Cerundolo, V. ; Guiraud, M. ; Muller, S. ; Valitutti, S.** Lytic versus Stimulatory synapse in cytotoxic T lymphocyte/target cell interaction: manifestation of a dual activation threshold. *Proc Natl Acad Sci U S A,* 2003, vol. 100, 14145-14150
- **Fukuda, M.** Lysosomal membrane glycoproteins, Structure, biosynthesis, and intracellular trafficking. *J Biol Chem,* 1991, vol. 266, 21327-21330
- **Gajewski, T. F. ; Goldwasser, E. ; Fitch, F. W.** Anti-proliferative effect of IFN-gamma in immune regulation. II. IFN-gamma inhibits the proliferation of murine bone marrow cells stimulated with IL-3, IL-4, or granulocyte-macrophage colony-stimulating factor. *J Immunol,* 1988, vol. 141, 2635-2642
- **Greenwood, J. ; Clark, M ; Waldmann, H.** Structural motifs involved in human IgG antibody effector functions. *Eur J Immunol,* 1993, vol. 23, 1098-1104
- **Hesslein, D. G. ; Takaki, R. ; Hermiston, M. L. ; Weiss, A. ; Lanier, L. L.** Dysregulation of signaling pathways in CD45-deficient NK cells leads to differentially regulated cytotoxicity and cytokine production. *Proc Natl Acad Sci U S A,* 2006, vol. 103, 7012-7017
- **Huntington, N. D. ; Xu, Y. ; Nutt, S. L. ; Tarlinton, D. M.** A requirement for CD45 distinguishes Ly49D-mediated cytokine and chemokine production from killing in primary natural killer cells. *J Exp Med,* 2005, vol. 201, 1421-1433
- **Kurago, Z. B. ; Lutz, C. T. ; Smith, K. D. ; Colonna, M.** NK cell natural cytotoxicity and IFN-gamma production are not always coordinately regulated: engagement of DX9 KIR+ NK cells by HLA-B7 variants and target cells. *J Immunol,* 1998, vol. 160, 1573-1580
- **Mendes, R. ; Bromelow, K. V. ; Westby, M. ; Galea-Lauri, J. ; Smith, I. E. ; O'Brien, M. E. ; Souberbielle, B. E.** Flow cytometric visualisation of cytokine production by CD3-CD56+ NK cells and CD3+CD56+ NK-T cells in whole blood. *Cytometry,* 2000, vol. 39, 72-78
- **Natarajan, K. ; Dimasi, N. ; Wang, J. ; Mariuzza, R. A. ; Margulies, D. H.** Structure and function of natural killer cell receptors: multiple molecular solutions to self, nonself discrimination. *Annu Rev Immunol,* 2002, vol. 20, 853-885
- **Nathan, C. F. ; Prendergast, T. J. ; Wiebe, M. E. ; Stanley, E. R. ; Platzer, E. ; Remold, H. G. ; Welte, K. ; Rubin, B. Y. ; Murray, H. W.** Activation of human macrophages. Comparison of other cytokines with interferon-gamma. *J Exp Med,* 1984, vol. 160, 600-605
- **Parihar, R. ; Dierksheide, J. ; Hu, Y. ; Carson, W. E.** IL-12 enhances the natural killer cell cytokine response to Ab-coated tumor cells. *J Clin Invest,* 2002, vol. 110, 983-992
- **Pende, D. ; Cantoni, C. ; Rivera, P. ; Vitale, M. ; Castriconi, R. ; Marcenaro, S. ; Nanni, M. ; Biassoni, R ; Bottino, C. ; Moretta, A.** Role of NKG2D in tumor cell lysis mediated by human NK cells: cooperation with natural cytotoxicity receptors and capability of recognizing tumors of nonepithelial origin. *Eur J Immunol,* 2001, vol. 31, 1076-1086
- **Rajagopalan, S. ; Fu, J. ; Long, E. O.** Cutting edge: induction of IFN-gamma production but not cytotoxicity by the killer cell Ig-like receptor KIR2DL4 (CD158d) in resting NK cells. *J Immunol,* 2001, vol. 167, 1877-1881
- **Reff, M. E. ; Camer, K. ; Chambers, K. S. ; Chinn, P. C. ; Leonard, J. E. ; Raab, R. ; Newman, R. A. ; Hanna, N. ; Anderson, D. R.** Depletion of B cells in vivo by a chimeric mouse human monoclonal antibody to CD20. *Blood,* 1994, vol. 83, 435-445
- **Shields, R. L. ; Namenuk, A. K. ; Hong, K. ; Meng, Y. G. ; Rae, J. ; Briggs, J. ; Xie, D. ; Lai, J. ; Stadlen, A. ; Li, B. et al.** High resolution mapping of the binding site on human IgGI for Fc gamma RI, Fc gamma RII, Fc gamma RIII, and FcRn and design of IgG1 variants with improved binding to the Fc gamma R. *J Biol Chem,* 2001, vol. 276, 6591-6604
- **Shinkawa, T. ; Nakamura, K. ; Yamane, N. ; Shoji-Hosaka, E. ; Kanda, Y. ; Sakurada, M. ; Uchida, K. ; Anazawa, H. ; Satoh, M. ; Yamasaki, M. et al.** The absence of fucose but not the presence of galactose or bisecting N-acetylglucosamine of human IgGI complex-type oligosaccharides shows the critical role of enhancing antibody-dependent cellular cytotoxicity. *J Biol Chem,* 2003, vol. 278, 3466-3473
- **Smyth, M. J. ; Cretney, E. ; Kelly, J. M. ; Westwood, J. A. ; Street, S. E. ; Yagita, H. ; Takeda, K. ; van Dommelen, S. L. ; Degli-Esposti, M. A. ; Hayakawa, Y.** Activation of NK cell cytotoxicity. *Mol Immunol,* 2005, vol. 42, 501-510
- **Trinchieri, G.** Biology of natural killer cells. *Adv Immunol,* 1989, vol. 47, 187-376
- **Umana, P. ; Jean-Mairet, J. ; Moudry, R. ; Amstutz, H. ; Bailey, J. E.** Engineered glycoforms of an antineuroblastoma IgG1 with optimized antibody-dependent cellular cytotoxic activity. *Nat Biotechnol,* 1999, vol. 17, 176-180
- **Vitale, M. ; Caruso, A. ; Licenziati, S. ; Rodella, L. ; Fiorentini, S. ; Zauli, G. ; Castelli, F. ; Manzoli, F. A. ; Turano, A.** Differential production of IFN-gamma, analyzed at the single-cell level, by specific subsets of human NK and T cells from healthy and HIV(+) subjects. *Cytometry,* 2000, vol. 39, 189-194
- **Wing, M. G. ; Moreau, T. ; Greenwood, J. ; Smith, R. M. ; Hale, G. ; Isaacs, J. ; Waldmann, H. ; Lachmann, P. J. ; Compston, A.** Mechanism of first-dose cytokine-release syndrome by CAMPATH 1-H: involvement of CD16 (FcgammaRIII) and CD11a/CD18 (LFA-1) on NK cells. *J Clin Invest,* 1996, vol. 98, 2819-2826